# EUROPEAN PATENT SPECIFICATION

(11) **EP 3 256 578 B1**
(45) Date of publication and mention of the grant of the patent: **12.02.2020**
(21) Application number: 16704213.4
(22) Date of filing: 12.02.2016
(51) Int. Cl.: C12N 9/22, C07K 14/005, C12N 15/62, A61K 48/00, C12N 15/867, C07K 19/00

(54) **POLYPEPTIDES FOR ENGINEERING INTEGRASE CHIMERIC PROTEINS AND THEIR USE IN GENE THERAPY**
POLYPEPTIDE ZUR ENTWICKLUNG CHIMÄRER INTEGRASEPROTEINE UND IHRE VERWENDUNG BEI DER GENTHERAPIE
POLYPEPTIDES POUR L'INGÉNIERIE DE PROTÉINES INTÉGRASES CHIMÉRIQUES ET LEUR UTILISATION EN THÉRAPIE GÉNIQUE

(30) Priority: 13.02.2015 EP 15305217
(43) Date of publication of application: 20.12.2017
(73) Proprietor: INSERM - Institut National de la Santé et de la Recherche Médicale, 75013 Paris (FR); Centre National de la Recherche Scientifique (C.N.R.S.), 75016 Paris (FR); Université Paris Diderot - Paris 7, 75013 Paris (FR); Commissariat à l'Energie Atomique et aux Energies Alternatives, 75015 Paris (FR); CNAM Conservatoire National des Arts des Metiers, 75003 Paris (FR)
(72) Inventor: BRIDIER-NAHMIAS, Antoine, 75010 Paris (FR); WERNER, Michel, 91191 Gif-sur-Yvette (FR); LESAGE, Pascale, 75010 Paris (FR)
(74) Representative: Inserm Transfert
(86) International application number: PCT/EP2016/053008
(87) International publication number: WO 2016/128549

(56) References cited:
- DEVINE, J. BOEKE: "Invading the yeast nucleus: a nuclear localization signal at the c terminus of ty1 integrase is required for transposition in vivo", MOL CELL BIOL, vol. 18, no. 2, February 1998 (1998-02), pages 1115-1124, XP002743549, cited in the application
- WILHELM MARCELLE ET AL: "Expression of an active form of recombinant Ty1 reverse transcriptase in Escherichia coli: A fusion protein containing the C-terminal region of the Ty1 integrase linked to the reverse transcriptase-RNase H domain exhibits polymerase and RNase H activities", BIOCHEMICAL JOURNAL, vol. 348, no. 2, 1 June 2000 (2000-06-01), pages 337-342, XP002743550, ISSN: 0264-6021
- S. P. MOORE; L. A. RINCKEL; D. J. GARFINKEL: "A Ty1 integrase nuclear localization signal required for retrotransposition", MOLECULAR AND CELLULAR BIOLOGY, vol. 18, no. 2, February 1998 (1998-02), pages 1105-1114, XP002743551, cited in the application
- MOORE SHARON P ET AL: "Expression and partial purification of enzymatically active recombinant Ty1 integrase in Saccharomyces cerevisiae", PROCEEDINGS OF THE NATIONAL ACADEMY OF SCIENCES OF THE UNITED STATES OF AMERICA, vol. 91, no. 5, 1994, pages 1843-1847, XP002743552, ISSN: 0027-8424
- MOORE SHARON P ET AL: "Substrate specificity of Ty1 integrase", JOURNAL OF VIROLOGY, vol. 69, no. 8, 1995, pages 4683-4692, XP002743553, ISSN: 0022-538X
- WILHELM M ET AL: "Role of integrase in reverse transcription of the Saccharomyces cerevisiae retrotransposon Ty1", EUKARYOTIC CELL, vol. 4, no. 6, June 2005 (2005-06), pages 1057-1065, XP002743554,
- OLIVIER DELELIS ET AL: "Integrase and integration: biochemical activities of HIV-1 integrase", RETROVIROLOGY, vol. 5, 17 December 2008 (2008-12-17), pages 1-13, DOI: doi: 10.1186/1742-4690-5-114
- ROBERT CRAIGIE: "The molecular biology of HIV integrase", FUTURE VIROL., vol. 7, no. 7, July 2012 (2012-07), pages 679-686, DOI: 10.2217/FVL.12.56

### SEQUENCE LISTING

<110> Inserm
<120> POLYPEPTIDES FOR ENGINEERING INTEGRASE CHIMERIC PROTEINS AND THEIR USE IN GENE THERAPY
<130> BIO 15039 LESAGE / MC
<160> 17
<170> PatentIn version 3.3
<210> 1
   <211> 635
   <212> PRT
   <213> Saccharomyces cerevisiae
<400> 1
   Asn Val His Thr Ser Glu Ser Thr Arg Lys Tyr Pro Tyr Pro Phe Ile
   1               5                   10                  15
   His Arg Met Leu Ala His Ala Asn Ala Gln Thr Ile Arg Tyr Ser Leu
               20                  25                  30
   Lys Asn Asn Thr Ile Thr Tyr Phe Asn Glu Ser Asp Val Asp Trp Ser
           35                  40                  45
   Ser Ala Ile Asp Tyr Gln Cys Pro Asp Cys Leu Ile Gly Lys Ser Thr
       50                  55                  60
   Lys His Arg His Ile Lys Gly Ser Arg Leu Lys Tyr Gln Asn Ser Tyr
   65                  70                  75                  80
   Glu Pro Phe Gln Tyr Leu His Thr Asp Ile Phe Gly Pro Val His Asn
                   85                  90                  95
   Leu Pro Asn Ser Ala Pro Ser Tyr Phe Ile Ser Phe Thr Asp Glu Thr
               100                 105                 110
   Thr Lys Phe Arg Trp Val Tyr Pro Leu His Asp Arg Arg Glu Asp Ser
           115                 120                 125
   Ile Leu Asp Val Phe Thr Thr Ile Leu Ala Phe Ile Lys Asn Gln Phe
       130                 135                 140
   Gln Ala Ser Val Leu Val Ile Gln Met Asp Arg Gly Ser Glu Tyr Thr
   145                 150                 155                 160
   Asn Arg Thr Leu His Lys Phe Leu Glu Lys Asn Gly Ile Thr Pro Cys
                   165                 170                 175
   Tyr Thr Thr Thr Ala Asp Ser Arg Ala His Gly Val Ala Glu Arg Leu
               180                 185                 190
   Asn Arg Thr Leu Leu Asp Asp Cys Arg Thr Gln Leu Gln Cys Ser Gly
           195                 200                 205
   Leu Pro Asn His Leu Trp Phe Ser Ala Ile Glu Phe Ser Thr Ile Val
       210                 215                 220
   Arg Asn Ser Leu Ala Ser Pro Lys Ser Lys Lys Ser Ala Arg Gln His
   225                 230                 235                 240
   Ala Gly Leu Ala Gly Leu Asp Ile Ser Thr Leu Leu Pro Phe Gly Gln
                   245                 250                 255
   Pro Val Ile Val Asn Asp His Asn Pro Asn Ser Lys Ile His Pro Arg
               260                 265                 270
   Gly Ile Pro Gly Tyr Ala Leu His Pro Ser Arg Asn Ser Tyr Gly Tyr
           275                 280                 285
   Ile Ile Tyr Leu Pro Ser Leu Lys Lys Thr Val Asp Thr Thr Asn Tyr
       290                 295                 300
   Val Ile Leu Gln Gly Lys Glu Ser Arg Leu Asp Gln Phe Asn Tyr Asp
   305                 310                 315                 320
   Ala Leu Thr Phe Asp Glu Asp Leu Asn Arg Leu Thr Ala Ser Tyr His
                   325                 330                 335
   Ser Phe Ile Ala Ser Asn Glu Ile Gln Glu Ser Asn Asp Leu Asn Ile
               340                 345                 350
   Glu Ser Asp His Asp Phe Gln Ser Asp Ile Glu Leu His Pro Glu Gln
           355                 360                 365
   Pro Arg Asn Val Leu Ser Lys Ala Val Ser Pro Thr Asp Ser Thr Pro
       370                 375                 380
   Pro Ser Thr His Thr Glu Asp Ser Lys Arg Val Ser Lys Thr Asn Ile
   385                 390                 395                 400
   Arg Ala Pro Arg Glu Val Asp Pro Asn Ile Ser Glu Ser Asn Ile Leu
                   405                 410                 415
   Pro Ser Lys Lys Arg Ser Ser Thr Pro Gln Ile Ser Asn Ile Glu Ser
               420                 425                 430
   Thr Gly Ser Gly Gly Met His Lys Leu Asn Val Pro Leu Leu Ala Pro
           435                 440                 445
   Met Ser Gln Ser Asn Thr His Glu Ser Ser His Ala Ser Lys Ser Lys
       450                 455                 460
   Asp Phe Arg His Ser Asp Ser Tyr Ser Glu Asn Glu Thr Asn His Thr
   465                 470                 475                 480
   Asn Val Pro Ile Ser Ser Thr Gly Gly Thr Asn Asn Lys Thr Val Pro
                   485                 490                 495
   Gln Ile Ser Asp Gln Glu Thr Glu Lys Arg Ile Ile His Arg Ser Pro
               500                 505                 510
   Ser Ile Asp Ala Ser Pro Pro Glu Asn Asn Ser Ser His Asn Ile Val
           515                 520                 525
   Pro Ile Lys Thr Pro Thr Thr Val Ser Glu Gln Asn Thr Glu Glu Ser
       530                 535                 540
   Ile Ile Ala Asp Leu Pro Leu Pro Asp Leu Pro Pro Glu Ser Pro Thr
   545                 550                 555                 560
   Glu Phe Pro Asp Pro Phe Lys Glu Leu Pro Pro Ile Asn Ser Arg Gln
                   565                 570                 575
   Thr Asn Ser Ser Leu Gly Gly Ile Gly Asp Ser Asn Ala Tyr Thr Thr
               580                 585                 590
   Ile Asn Ser Lys Lys Arg Ser Leu Glu Asp Asn Glu Thr Glu Ile Lys
           595                 600                 605
   Val Ser Arg Asp Thr Trp Asn Thr Lys Asn Met Arg Ser Leu Glu Pro
       610                 615                 620
   Pro Arg Ser Lys Lys Arg Ile His Leu Ile Ala
   625                 630                 635
<210> 2
   <211> 6
   <212> PRT
   <213> Artificial
<220>
   <223> interaction domain with AC40
<400> 2
   Lys Asn Met Arg Ser Leu
   1 5
<210> 3
   <211> 6
   <212> PRT
   <213> Artificial
<220>
   <223> interaction domain with AC40
<400> 3
   Lys Glu Met Asp Ser Leu
   1 5
<210> 4
   <211> 7
   <212> PRT
   <213> Artificial
<220>
   <223> interaction domain wiht AC40
<400> 4
   Lys Asn Met Arg Ser Leu Glu
   1 5
<210> 5
   <211> 7
   <212> PRT
   <213> Artificial
<220>
   <223> interaction domain with AC40
<400> 5
   Lys Ala Met Arg Ser Leu Glu
   1 5
<210> 6
   <211> 7
   <212> PRT
   <213> Artificial
<220>
   <223> interaction domain wiht AC40
<400> 6
   Lys Asn Met Arg Ser Leu Ala
   1 5
<210> 7
   <211> 9
   <212> PRT
   <213> Artificial
<220>
   <223> interaction domain wiht AC40
<400> 7
   Lys Asn Met Arg Ser Leu Glu Pro Pro
   1               5
<210> 8
   <211> 15
   <212> PRT
   <213> Artificial
<220>
   <223> interaction domain with AC40
<400> 8
   Val Ser Arg Asp Thr Trp Asn Thr Lys Asn Met Arg Ser Leu Glu
   1               5                   10                  15
<210> 9
   <211> 27
   <212> PRT
   <213> Artificial
<220>
   <223> interaction domain with AC40
<400> 9
   Ser Leu Glu Asp Asn Glu Thr Glu Ile Lys Val Ser Arg Asp Thr Trp
   1               5                   10                  15
   Asn Thr Lys Asn Met Arg Ser Leu Glu Pro Pro
               20                  25
<210> 10
   <211> 36
   <212> PRT
   <213> Artificial
<220>
   <223> interaction domain with AC40
<400> 10
   Ser Lys Lys Arg Ser Leu Glu Asp Asn Glu Thr Glu Ile Lys Val Ser
   1 5 10 15
   Arg Asp Thr Trp Asn Thr Lys Asn Met Arg Ser Leu Glu Pro Pro Arg
   20 25 30
   Ser Lys Lys Arg
   35
<210> 11
   <211> 41
   <212> PRT
   <213> Artificial
<220>
   <223> interaction domain wiht AC40
<400> 11
   Ser Lys Lys Arg Ser Leu Glu Asp Asn Glu Thr Glu Ile Lys Val Ser
   1 5 10 15
   Arg Asp Thr Trp Asn Thr Lys Asn Met Arg Ser Leu Glu Pro Pro Arg
   20 25 30
   Ser Lys Lys Arg Ile His Leu Ile Ala
   35 40
<210> 12
   <211> 58
   <212> PRT
   <213> Artificial
<220>
   <223> interaction domain wiht AC40
<400> 12
   Asn Ser Ser Leu Gly Gly Ile Gly Asp Ser Asn Ala Tyr Thr Thr Ile
   1 5 10 15
   Asn Ser Lys Lys Arg Ser Leu Glu Asp Asn Glu Thr Glu Ile Lys Val
   20 25 30
   Ser Arg Asp Thr Trp Asn Thr Lys Asn Met Arg Ser Leu Glu Pro Pro
   35 40 45
   Arg Ser Lys Lys Arg Ile His Leu Ile Ala
   50 55
<210> 13
   <211> 288
   <212> PRT
   <213> Human immunodeficiency virus
<400> 13
   Phe Leu Asp Gly Ile Asp Lys Ala Gln Asp Glu His Glu Lys Tyr His
   1               5                   10                  15
   Ser Asn Trp Arg Ala Met Ala Ser Asp Phe Asn Leu Pro Pro Val Val
   20 25 30
   Ala Lys Glu Ile Val Ala Ser Cys Asp Lys Cys Gln Leu Lys Gly Glu
   35 40 45
   Ala Met His Gly Gln Val Asp Cys Ser Pro Gly Ile Trp Gln Leu Asp
   50 55 60
   Cys Thr His Leu Glu Gly Lys Val Ile Leu Val Ala Val His Val Ala
   65 70 75 80
   Ser Gly Tyr Ile Glu Ala Glu Val Ile Pro Ala Glu Thr Gly Gln Glu
   85 90 95
   Thr Ala Tyr Phe Leu Leu Lys Leu Ala Gly Arg Trp Pro Val Lys Thr
   100 105 110
   Ile His Thr Asp Asn Gly Ser Asn Phe Thr Gly Ala Thr Val Arg Ala
   115 120 125
   Ala Cys Trp Trp Ala Gly Ile Lys Gln Glu Phe Gly Ile Pro Tyr Asn
   130 135 140
   Pro Gln Ser Gln Gly Val Val Glu Ser Met Asn Lys Glu Leu Lys Lys
   145 150 155 160
   Ile Ile Gly Gln Val Arg Asp Gln Ala Glu His Leu Lys Thr Ala Val
   165 170 175
   Gln Met Ala Val Phe Ile His Asn Phe Lys Arg Lys Gly Gly Ile Gly
   180 185 190
   Gly Tyr Ser Ala Gly Glu Arg Ile Val Asp Ile Ile Ala Thr Asp Ile
   195 200 205
   Gln Thr Lys Glu Leu Gln Lys Gln Ile Thr Lys Ile Gln Asn Phe Arg
   210 215 220
   Val Tyr Tyr Arg Asp Ser Arg Asn Pro Leu Trp Lys Gly Pro Ala Lys
   225 230 235 240
   Leu Leu Trp Lys Gly Glu Gly Ala Val Val Ile Gln Asp Asn Ser Asp
   245 250 255
   Ile Lys Val Val Pro Arg Arg Lys Ala Lys Ile Ile Arg Asp Tyr Gly
   260 265 270
   Lys Gln Met Ala Gly Asp Asp Cys Val Ala Ser Arg Gln Asp Glu Asp
   275 280 285
<210> 14
   <211> 407
   <212> PRT
   <213> Moloney murine sarcoma virus
<400> 14
   Glu Asn Ser Ser Pro Tyr Thr Ser Glu His Phe His Tyr Thr Val Thr
   1 5 10 15
   Asp Ile Lys Asp Leu Thr Lys Leu Gly Ala Ile Tyr Asp Lys Thr Lys
   20 25 30
   Lys Tyr Trp Val Tyr Gln Gly Lys Pro Val Met Pro Asp Gln Phe Thr
   35 40 45
   Phe Glu Leu Leu Asp Phe Leu His Gln Leu Thr His Leu Ser Phe Ser
   50 55 60
   Lys Met Lys Ala Leu Leu Glu Arg Ser His Ser Pro Tyr Tyr Met Leu
   65 70 75 80
   Asn Arg Asp Arg Thr Leu Lys Asn Ile Thr Glu Thr Cys Lys Ala Cys
   85 90 95
   Ala Gln Val Asn Ala Ser Lys Ser Ala Val Lys Gln Gly Thr Arg Val
   100 105 110
   Arg Gly His Arg Pro Gly Thr His Trp Glu Ile Asp Phe Thr Glu Ile
   115 120 125
   Lys Pro Gly Leu Tyr Gly Tyr Lys Tyr Leu Leu Val Phe Ile Asp Thr
   130 135 140
   Phe Ser Gly Trp Ile Glu Ala Phe Pro Thr Lys Lys Glu Thr Ala Lys
   145 150 155 160
   Val Val Thr Lys Lys Leu Leu Glu Glu Ile Phe Pro Arg Phe Gly Met
   165 170 175
   Pro Gln Val Leu Gly Thr Asp Asn Gly Pro Ala Phe Val Ser Lys Val
   180 185 190
   Ser Gln Thr Val Ala Asp Leu Leu Gly Ile Asp Trp Lys Leu His Cys
   195 200 205
   Ala Tyr Arg Pro Gln Ser Ser Gly Gln Val Glu Arg Met Asn Arg Thr
       210                 215                 220
   Ile Lys Glu Thr Leu Thr Lys Leu Thr Leu Ala Thr Gly Ser Arg Asp
   225                 230                 235                 240
   Trp Val Leu Leu Leu Pro Leu Ala Leu Tyr Arg Ala Arg Asn Thr Pro
                   245                 250                 255
   Gly Pro His Gly Leu Thr Pro Tyr Glu Ile Leu Tyr Gly Ala Pro Pro
               260                 265                 270
   Pro Leu Val Asn Phe Pro Asp Pro Asp Met Thr Arg Val Thr Asn Ser
           275                 280                 285
   Pro Ser Leu Gln Ala His Leu Gln Ala Leu Tyr Leu Val Gln His Glu
       290                 295                 300
   Val Trp Arg Pro Leu Ala Ala Ala Tyr Gln Glu Gln Leu Asp Arg Pro
   305                 310                 315                 320
   Val Val Pro His Pro Tyr Arg Val Gly Asp Thr Val Trp Val Arg Arg
                   325                 330                 335
   His Gln Thr Lys Asn Leu Glu Pro Arg Trp Lys Gly Pro Tyr Thr Val
               340                 345                 350
   Leu Leu Thr Thr Pro Thr Ala Leu Lys Val Asp Gly Ile Ala Ala Trp
           355                 360                 365
   Ile His Ala Ala His Val Lys Ala Ala Asp Pro Gly Gly Gly Pro Ser
       370                 375                 380
   Ser Arg Leu Thr Trp Arg Val Gln Arg Ser Gln Asn Pro Leu Lys Ile
   385                 390                 395                 400
   Arg Leu Thr Arg Glu Ala Pro
                   405
<210> 15
   <211> 21
   <212> PRT
   <213> Artificial
<220>
   <223> Ty5 interacting domain
<400> 15
   Glu Ser Pro Pro Ser Leu Asp Ser Ser Pro Pro Asn Thr Ser Phe Asn
   1 5 10 15
   Ala Ala Leu Thr Ala
   20
<210> 16
   <211> 27
   <212> PRT
   <213> Artificial
<220>
   <223> Ty4 interacting domain
<400> 16
   Ile Glu Ala Ser Gly Ser Pro Val Gln Thr Val Asn Lys Ser Ala Phe
   1 5 10 15
   Leu Asn Lys Glu Phe Ser Ser Leu Asn Met Lys
   20 25
<210> 17
   <211> 9
   <212> PRT
   <213> Artificial
<220>
   <223> interaction domain wiht AC40
<400> 17
   Trp Asn Thr Lys Asn Met Arg Ser Leu
   1 5

## Description

### FIELD:

The present disclosure relates to polypeptide for engineering integrase chimeric proteins and their use in gene therapy.

### BACKGROUND:

The capacity to introduce a particular foreign or native gene sequence into a cell and to control the expression of that gene is of value in the fields of medicine and biological research. The introduction of a particular foreign or native gene into a host cell can be facilitated by introducing a gene sequence into a suitable nucleic acid vector. A variety of methods have been developed that allow the introduction of such a recombinant vector into a desired host cell. The use of viral vectors can result in the rapid introduction of the recombinant molecule into a wide variety of host cells. For instance, retroviruses are RNA viruses that replicate through a DNA proviral intermediate that is usually integrated in the genome of the infected host cell. All known retroviruses share features of the replicative cycle, including packaging of viral RNA into virions, entry into target cells, reverse transcription of viral RNA to form the DNA provirus, and stable integration of the provirus into the target cell genome. Replication competent simple proviruses typically comprise regulatory long terminal repeats (LTRs) and the gag, pro, pol and env genes which encode core proteins (gag), a protease (pro), reverse transcriptase (pol), RNAse H (pol), integrase (pol) and envelope glycoproteins (env). Complex retroviruses also typically comprise additional accessory genes. Retroviral vectors are a common tool for gene delivery in that the ability of retroviral vectors to deliver an unrearranged, single copy gene into a broad range of cells makes them well suited for transferring genes to a cell. While recombinant retroviral vectors allow for integration of a transgene into a host cell genome, most retroviruses can only transduce dividing cells. This can limit their use for in vivo gene transfer to non-proliferating cells such as hepatocytes, myofibers, hematopoietic stem cells (HSCs), and neurons. Non-dividing cells are the predominant, long-lived cell type in the body, and account for most desirable targets of gene transfer, including liver, muscle, and brain. Lentiviruses are a subgroup of retroviruses that are capable of infecting non-dividing cells. These viruses include, but are not limited to, HIV-1, HIV-2, SIV, EIAV1 and FIV. Lentiviruses possess gag, pol, and env genes in addition to other accessory genes that are flanked by two long terminal repeat (LTR) sequences. A key challenge for gene transfer based on the use of retroviral vectors is to achieve stable transgene expression while minimizing insertional mutagenesis and induction of the DNA damage response due to the presence of double stranded DNA. One approach to avoid insertional mutagenesis is to target the transgene integration to a specific location on the genome.

Some mutants of mutants of Ty1 integrase have been already described (Kenna, M. A., Brachmann, C. B., Devine, S. E., & Boeke, J. D. (1998). Invading the yeast nucleus: a nuclear localization signal at the C terminus of Ty1 integrase is required for transposition in vivo. Molecular and Cellular Biology, 18(2), 1115-1124).

Furthermore, Delelis O. et al. disclose HIV integrase (Delelis, O., Carayon, K., Saïb, A., Deprez, E., & Mouscadet, J. F. (2008). Integrase and integration: biochemical activities of HIV-1 integrase. Retrovirology, 5(1), 114*).*

### SUMMARY OF THE PRESENT INVENTION:

As defined by the claims, the present invention relates to:
- a polypeptide which consists of the amino acid sequence selected from the group consisting of SEQ ID NO:2 to SEQ ID NO:12,
- a chimeric integrase which comprises the polypeptide of the present invention and which is selected from the group consisting of feline immunodeficiency virus (FIV) integrases, Foamy virus (FV) integrases, murine leukemia virus (MLV) integrases, and lentivirus integrases,
- a nucleic acid molecule which encodes for the polypeptide of the present invention or the chimeric integrase of the present invention,
- a retroviral vector which comprises the chimeric integrase of the present invention,
- An in vitro method for expressing a transgene into the genome of a plurality of cells comprising infecting the plurality of cells with the retroviral vector of the present invention, and
- the retroviral vector of the present invention for use in gene therapy.

### DETAILED DESCRIPTION:

Mobile genetic elements are ubiquitous. Their integration site influences genome stability and gene expression. The Ty1 retrotransposon of the yeast *Saccharomyces cerevisiae* integrates upstream of RNA polymerase III (Pol III)-transcribed genes, yet, the primary determinant of target specificity has remained elusive. Here, the inventors describe an interaction between Ty1 integrase and the AC40 subunit of Pol III, and demonstrate that AC40 is the predominant determinant of targeting Ty1 integration upstream of Pol III-transcribed genes. Lack of an integrase/AC40 interaction dramatically alters target site choice, leading to a redistribution of Ty1 insertions in the genome, principally to chromosome ends. The mechanism of target specificity to individually non-essential genes allows Ty1 to proliferate and yet minimizes genetic damage to its host. Accordingly, the domain of Ty1 responsible for the interaction with the RNA polymerase III could thus be suitable to engineering integrase of retrovirus so as to drive the targeted integration of a transgene into a eukaryotic genome.

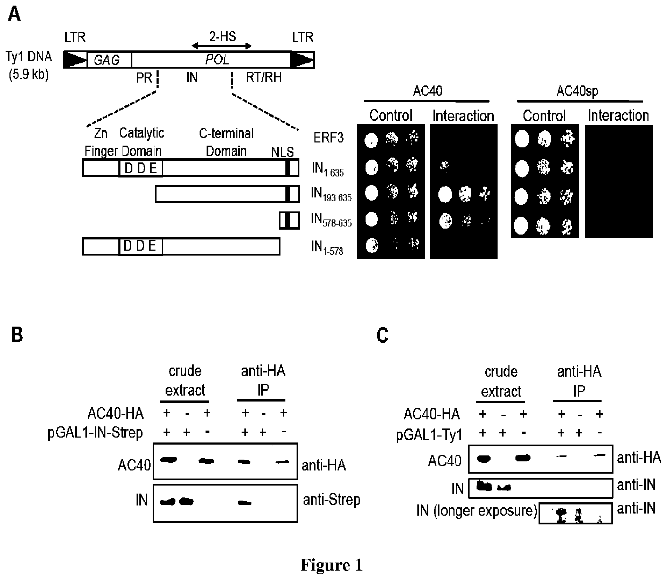

As used herein, the term "Ty1 integrase" or "Ty1 IN" has its general meaning in the art and refers to the Ty1 retroelement's encoded integrase. Ty1, the most active and abundant LTR-retrotransposon of S. cerevisiae, preferentially integrates within a 1-kb window upstream of Pol III-transcribed genes. Ty1 IN has two phylogenetically conserved regions (an N-terminal zinc-binding domain and a catalytic core with the DDX35E motif) and a less conserved C-terminus containing a bi-partite nuclear localization signal required for IN nuclear localization and efficient retrotransposition (Figure 1A) (M. Kenna, C. Brachmann, S. Devine, J. Boeke, Mol Cell Biol 18, 1115-1124 (1998).; 9. S. P. Moore, L. A. Rinckel, D. J. Garfinkel, Mol Cell Biol 18, 1105-1114 (1998)). An exemplary amino acid sequence of Ty1 integrase is represented by SEQ ID NO:1:

A first object of the present disclosure thus relates to a polypeptide which comprises the amino acid sequence ranging from the amino acid residue at position 617 to the amino acid residue at position 622 in SEQ ID NO:1 (KNMRSL = SEQ ID NO:2) or a function conservative thereof.

As used herein the term "function-conservative variants" are those in which a given amino acid residue in the polypeptide has been changed without altering the overall conformation and function (i.e. interaction with AC40) of the polypeptide, including, but not limited to, replacement of an amino acid with one having similar properties (conservative substitution) or replacement of an amino acid with one having different properties (non conservative substitution). Amino acids other than those indicated as conserved may differ in the polypeptide so that the percent of identity between any two polypeptides of similar function may vary and may be, for example, from 70 % to 99 % as determined according to an alignment scheme such as by the Cluster Method, wherein similarity is based on the MEGALIGN algorithm. A "function-conservative variant" also includes a polypeptide having an amino acid sequence having at least 50% of identity with the sequence ranging from the amino acid residue at position 617 to the amino acid residue at position 622 in SEQ ID NO: 1. In particular, the function-conservative variant comprises an amino acid sequence having 50; 51; 52; 53; 54; 55; 56; 57; 58; 59; 60; 61; 62; 63; 64; 65; 66; 67; 68; 69; 70; 71; 72; 73; 74; 75; 76; 77; 78; 79; 80; 81; 82; 83; 84; 85; 86; 87; 88; 89; 90; 91; 92; 93; 94; 95; 96; 97; 98; 99% of identity with the amino acid sequence ranging from the amino acid residue at position 617 to the amino acid residue at position 622 in SEQ ID NO:1.

As used herein, an "amino acid residue" refers to any naturally occurring amino acid, any amino acid derivative or any amino acid mimic known in the art. In particular, the residues of the protein or peptide are sequential, without any non-amino acid interrupting the sequence of amino acid residues. In other embodiments, the sequence may comprise one or more non-amino acid moieties. In particular embodiments, the sequence of residues of the protein or peptide may be interrupted by one or more non-amino acid moieties. Accordingly, the term protein or peptide encompasses amino acid sequences comprising at least one of the 20 common amino acids found in naturally occurring proteins, or at least one modified or unusual amino acid, including, but not limited to, 2-Aminoadipic acid (Aad), N-Ethylasparagine (EtAsn), 3-Aminoadipic acid (Baad), Hydroxylysine (Hyl), β alanine, β Amino propionic acid (Bala), allo Hydroxylysine (AHyl), 2-Aminobutyric acid (Abu), 3-Hydroxyproline (3Hyp), 4-Aminobutyric acid (4Abu), 4-Hydroxyproline (4Hyp), 6-Aminocaproic acid (Acp), Isodesmosine (Ide), 2-Aminoheptanoic acid (Ahe), allo Isoleucine (AIle), 2-Aminoisobutyric acid (Aib), N-Methylglycine (MeGly), 3-Aminoisobutyric acid (Baib), N-Methylisoleucine (Melle), 2-Aminopimelic acid (Apm), 6-N-Methyllysine (MeLys), 2,4-Diaminobutyric acid (Dbu), N-Methylvaline (MeVal), Desmosine (Des), Norvaline (Nva), 2,2'-Diaminopimelic acid (Dpm), Norleucine (Nle), 2,3-Diaminopropionic acid (Dpr), Ornithine (Orn), or N-Ethylglycine (EtGly).

In particular, the amino acid residue at position 617, 619, 621 or 622 is conservatively substituted. In the context of the present disclosuredisclosure, a "conservative substitution" is defined by substitutions within the classes of amino acids reflected as follows:
Aliphatic residues I, L, V, and M
Cycloalkenyl-associated residues F, H, W, and Y
Hydrophobic residues A, C, F, G, H, I, L, M, R, T, V, W, and Y
Negatively charged residues D and E
Polar residues C, D, E, H, K, N, Q, R, S, and T
Positively charged residues H, K, and R
Small residues A, C, D, G, N, P, S, T, and V
Very small residues A, G, and S
Residues involved in turn A, C, D, E, G, H, K, N, Q, R, S, P, and formation T
Flexible residues Q, T, K, S, G, P, D, E, and R

More conservative substitutions groupings include: valine-leucine-isoleucine, phenylalanine-tyrosine, lysine-arginine, alanine-valine, and asparagine-glutamine. Conservation in terms of hydropathic/hydrophilic properties and residue weight/size also is substantially retained in the polypeptide of the present disclosure as compared to the native sequence. The importance of the hydropathic amino acid index in conferring interactive biologic function on a protein is generally understood in the art. It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathical index on the basis of their hydrophobicity and charge characteristics these are: isoleucine (+4.5); valine (+4.2); leucine (+3.8) ; phenylalanine (+2.8); cysteine/cystine (+2.5); methionine (+1.9); alanine (+1.8); glycine (-0.4); threonine (-0.7); serine (-0.8); tryptophane (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5). The retention of similar residues may also or alternatively be measured by a similarity score, as determined by use of a BLAST program (e.g., BLAST 2.2.8 available through the NCBI using standard settings BLOSUM62, Open Gap= 11 and Extended Gap= 1).

In particular, the amino acid a position 618 or 620 is substituted by any amino acid (i.e. a non conservative substitution). In particular, the amino acid residue at position 618 is substituted by an alanine (A). In particular, the amino acid residue at position 620 is substituted by an aspartic acid residue (D).

In particular, the polypeptide of the present disclosure comprises the amino acid sequence as set forth in SEQ ID NO:3 (K**E**M**D**SL).

In particular, the polypeptide of the present disclosure comprises 6; 7; 8; 9; 10; 11; 12; 13; 14; 15; 16; 17; 18; 19; 20; 21; 22; 23; 24; 25; 26; 27; 28; 29; 30; 31; 32; 33; 34; 35; 36; 37; 38; 39; 40; 41; 42; 43; 44; 45; 46; 47; 48; 49; 50; 51; 52; 53; 54; 55; 56; 57; 58; 59; 60; 61; 62; 63; 64; 65; 66; 67; 68; 69 ; 70; 71; 72; 73; 74; 75; 76; 77; 78; 79; 80; 81; 82; 83; 84; 85; 86; 87; 88; 89; 90; 91; 92; 93; 94; 95; 96; 97; 98; 99; 100; 101; 102; 103; 104; 105; 106; 107; 108; 109; 110; 111; 112; 113; 114; 115; 116; 117; 118; 119; 120; 121; 122; 123; 124; 125; 126; 127; 128; 129; 130; 131; 132; 133; 134; 135; 136; 137; 138; 139; 140; 141; 142; 143; 144; 145; 146; 147; 148; 149; 150; 151; 152; 153; 154; 155; 156; 157; 158; 159; 160; 161; 162; 163; 164; 165; 166; 167; 168; 169; 170; 171; 172; 173; 174; 175; 176; 177; 178; 179; 180; 181; 182; 183; 184; 185; 186; 187; 188; 189; 190; 191; 192; 193; 194; 195; 196; 197; 198; 199; or 200 amino acid residues. In particular, the polypeptide of the present disclosure comprises 7;6; 8; 9; 10; 11; 12; 13; 14; 15; 16; 17; 18; 19; 20; 21; 22; 23; 24; 25; 26; 27; 28; 29; 30; 31; 32; 33; 34; 35; 36; 37; 38; 39; 40; 41; 42; 43; 44; 45; 46; 47; 48; 49; 50; 51; 52; 53; 54; 55; 56; 57; 58; 59 or 60 consecutive amino acid residues in SEQ ID NO:1.

In particular, the polypeptide of the present disclosure comprises the amino acid sequence ranging from the amino acid residue at position 617 to the amino acid residue at position 623 in SEQ ID NO:1 (KNMRSLE = SEQ ID NO: 4).

In particular, the polypeptide of the present disclosure comprises the amino acid sequence as set forth in SEQ ID NO:5 (KAMRSLE).

In particular, the polypeptide of the present disclosure comprises the amino acid sequence as set forth in SEQ ID NO:6 (KNMRSLA).

In particular, the polypeptide of the present disclosure comprises the amino acid sequence ranging from the amino acid residue at position 617 to the amino acid residue at position 625 (KNMRSLEPP = SEQ ID NO:7).

In particular, the polypeptide of the present disclosure comprises the amino acid sequence ranging from the amino acid residue at position 614 to the amino acid residue at position 622 in SEQ ID NO:1 (WNTKNMRSL = SEQ ID NO: 17).

In particular, the polypeptide of the present disclosure comprises the amino acid sequence ranging from the amino acid residue at position 609 to the amino acid residue at position 623 (VSRDTWNTKNMRSLE = SEQ ID NO:8).

In particular, the polypeptide of the present disclosure comprises the amino acid sequence ranging from the amino acid residue at position 600 to the amino acid residue at position 625 (SLEDNETEIKVSRDTWNTKNMRSLEPP = SEQ ID NO: 9).

In particular, the polypeptide of the present disclosure comprises the amino acid sequence ranging from the amino acid residue at position 595 to the amino acid residue at position 630 (SKKRSLEDNETEIKVSRDTWNTKNMRSLEPPRSKKR = SEQ ID NO:10).

In particular, the polypeptide of the present disclosure comprises the amino acid sequence ranging from the amino acid residue at position 595 to the amino acid residue at position 635 (SKKRSLEDNETEIKVSRDTWNTKNMRSLEPPRSKKRIHLIA = SEQ ID NO: 11).

In particular, the polypeptide of the present disclosure comprises the amino acid sequence ranging from the amino acid residue at position 578 to the amino acid residue at position 635 (NSSLGGIGDSNAYTTINSK KRSLEDNETEIKVSRDTWNTKNMRSLEPPRSKKRIHLIA = SEQ ID NO:12).

In particular, the polypeptide of the present disclosure comprises the amino acid sequence selected from the group consisting of SEQ ID NO:2 to SEQ ID NO:12 and amino acid sequences having at least 50% of identity with SEQ ID NO:2 to SEQ ID NO:12.

In particular, the polypeptide of the present disclosure is fused to a heterologous polypeptide (i.e. a polypeptide that is not derived from the native amino sequence of Ty1 integrase) to form a fusion protein. As used herein, a "fusion protein" comprises all or part (typically biologically active) of a polypeptide of the present disclosure operably linked to a heterologous polypeptide (i.e., a polypeptide other than the same polypeptide). Within the fusion protein, the term "operably linked" is intended to indicate that the polypeptide of the present disclosure and the heterologous polypeptide are fused in-frame to each other. The heterologous polypeptide can be fused to the N-terminus or C-terminus of the polypeptide of the present disclosure. In some embodiment, the heterologous polypeptide is fused to the C-terminal end of the polypeptide of the present disclosure. In particular, the polypeptide of the present disclosure and the heterologous polypeptide are fused to each other directly (i.e. without use of a linker) or via a linker. The linker is typically a linker peptide and will, of the present disclosure, be selected so as to allow binding of the polypeptide to the heterologous polypeptide. Suitable linkers will be clear to the skilled person based on the disclosure herein, optionally after some limited degree of routine experimentation. Suitable linkers are described herein and may - for example and without limitation - comprise an amino acid sequence, which amino acid sequence preferably has a length of 2 or more amino acids. Typically, the linker has 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acids. However, the upper limit is not critical but is chosen for reasons of convenience regarding e.g. biopharmaceutical production of such fusion proteins. The linker sequence may be a naturally occurring sequence or a non-naturally occurring sequence. If used for therapeutical purposes, the linker is preferably non-immunogenic in the subject to which the fusion protein of the present disclosure is administered. One useful group of linker sequences are linkers derived from the hinge region of heavy chain antibodies as described in WO 96/34103 and WO 94/04678. Other examples are poly-alanine linker sequences such as Ala-Ala-Ala. Further preferred examples of linker sequences are Gly/Ser linkers of different length including (gly4ser)3 , (gly4ser)4, (gly4ser), (gly3ser), gly3, and (gly3ser2)3.

A further aspect of the present disclosure relates to a chimeric integrase wherein a retroviral integrase comprises a polypeptide of the present disclosure. According to the disclosure the chimeric integrase of the present disclosure is particularly suitable for directing the integration away from genomic sites that may prove detrimental to the cell or the organism harboring the cell. In particular, a recombinant integrase (IN) is thus engineered to contain a polypeptide of the present disclosure.

As used herein the term "retroviral integrase" has its general meaning in the art and refers to an enzyme produced by a retrovirus (such as HIV) that enables its genetic material to be integrated into the DNA of the infected cell. The main function of IN is to insert the viral DNA into the host chromosomal DNA, a step that is essential for HIV replication. Integration is a point of no return for the cell, which becomes a permanent carrier of the viral genome (provirus). All retroviral IN proteins have three physically distinct domains. (1) An N-terminal domain that includes three-helices and a zinc-binding motif. This domain has been implicated in dimerization and in binding the LTR ends. (2) The central domain that contains the conserved catalytic triad DDE. (3) The C-terminal domain that contributes to oligomerization and has nonspecific DNA-binding activity. The term "retrovirus" includes, but is not limited to, the members of the family retroviridae, including alpharetroviruses (e.g., avian leukosis virus), betaretroviruses (e.g., mouse mammary tumor virus), gammaretroviruses (e.g., murine leukemia virus), deltaretroviruses (e.g., bovine leukemia virus), epsilonretroviruses (e.g., Walley dermal sarcoma virus), lentiviruses (e.g., HIV-1, HIV-2) and spumaviruses (e.g., human spumavirus). The term "lentivirus" as used herein, refers to human immunodeficiency virus-1 (HIV-1); human immunodeficiency virus-2 (HIV-2); simian immunodeficiency virus (SIV); and feline immunodeficiency virus (FIV).

In particular, the retroviral integrase is a feline immunodeficiency virus (FIV) integrase, a Foamy virus (FV) integrase, a murine leukemia virus (MLV) integrase, a lentivirus integrase or other virally encoded integrase, or derivative thereof.

In particular, the retroviral integrase has a sequence having at least 80% of identity with SEQ ID NO: 13 or SEQ ID NO:14.

In particular, the integrase protein is mutated in the manner that their interaction with its cognate tethering factor is abolished. In particular, the integrase of SEQ ID NO: 13 comprises at least one mutation selected from the group consisting of V165A, R166A, L172A/K173A, Q168A, and Q168L. In particular, the integrase of SEQ ID NO: 14 comprises a mutation at position W390P398, L399, K400, R402 and in particular comprises the mutation W390A (Mol Ther Nucleic Acids. 2014 Jul; 3(7): e179.).

In particular, the polypeptide of the present disclosure is fused at the C-terminal end of the integrase protein. In particular, the polypeptide of the present disclosure is fused at the N-terminal end of the integrase protein. In particular, the polypeptide of the present disclosure is inserted in the sequence encoding for the integrase protein.

In particular, the polypeptide of the present disclosure is inserted between the amino acid residue at position 5 and the amino acid residue at position 6 in SEQ ID NO: 13.

In particular, the polypeptide of the present disclosure the amino acid residue at position 390 in SEQ ID NO:3 is replaced by the polypeptide of the present disclosure. In particular, the polypeptide of the present disclosure is inserted between the amino acid residue at position 389 and the amino acid residue at position 390 in SEQ ID NO:14 wherein said amino acid residue at position 390 is substituted (e.g. W390A).

A further object of the present disclosure relates to a nucleic acid molecule which encodes for a polypeptide of the present disclosure or a chimeric integrase of the present disclosure.

As used herein, the term "nucleic acid molecule" has its general meaning in the art and refers to a DNA or RNA molecule. However, the term captures sequences that include any of the known base analogues of DNA and RNA such as, but not limited to 4-acetylcytosine, 8-hydroxy-N6-methyladenosine, aziridinylcytosine, pseudoisocytosine, 5-(carboxyhydroxylmethyl) uracil, 5-fiuorouracil, 5-bromouracil, 5-carboxymethylaminomethyl-2-thiouracil, 5-carboxymethyl-aminomethyluracil, dihydrouracil, inosine, N6-isopentenyladenine, 1 -methyladenine, 1 -methylpseudouracil, 1-methylguanine, 1- methylinosine, 2,2-dimethylguanine, 2-methyladenine, 2-methylguanine, 3-methylcytosine, 5- methylcytosine, N6-methyladenine, 7-methylguanine, 5-methylaminomethyluracil, 5-methoxyamino-methyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, -uracil-5-oxyacetic acid methylester, uracil-5-oxyacetic acid, pseudouracil, queosine, 2-thiocytosine, and 2,6-diaminopurine.

In particular, the chimeric integrase of the present disclosure is packaged in a vector, more preferably a retroviral vector. Accordingly, a further object of the present disclosure relates to a retroviral vector (e.g. a lentiviral vector) which comprises a chimeric integrase of the present disclosure.

The term "retroviral vector" refers to a vector containing structural and functional genetic elements that are primarily derived from a retrovirus.

In particular, the retroviral vector of the present disclosure derives from a retrovirus selected from the group consisting of alpharetroviruses (e.g., avian leukosis virus), betaretroviruses (e.g., mouse mammary tumor virus), gammaretroviruses (e.g., murine leukemia virus), deltaretroviruses (e.g., bovine leukemia virus), epsilonretroviruses (e.g., Walley dermal sarcoma virus), lentiviruses (e.g., HIV-1, HIV-2) and spumaviruses (e.g., human spumavirus).

In particular, the retroviral vector of the present disclosure is a replication deficient retroviral virus particle, which can transfer a foreign imported RNA of a gene or a fragment thereof or a reporter gene, e.g. a therapeutic gene, instead of the retroviral mRNA.

In particular, the retroviral vector of the present disclosure is a lentiviral vector. The term "lentiviral vector" refers to a vector containing structural and functional genetic elements outside the LTRs that are primarily derived from a lentivirus. In particular, the lentiviral vector of the present disclosure is selected from the group consisting of HIV-1, HIV-2, SIV, FIV, EIAV, BIV, VISNA and CAEV vectors. In particular, the lentiviral vector is a HIV-1 vector.

In particular, the envelope protein of the retroviral vector of the present disclosure is pseudotyped with the envelope protein of the retrovirus used to prepare the retroviral vector, or alternatively with a heterogeneous envelope protein that is chosen with respect to the cells to be targeted into the host. In particular, the retroviral vector of the present disclosure is pseudotyped with a VSV-G protein. The VSV-G glycoprotein may originate from different serotypes of the genus of the vesiculoviruses: VSV-Indiana serotype, VSV-New Jersey serotype or other glycoproteins of the vesiculoviruses such as Piry, Chandipura, Isfahan and Cocal. The VSV-G glycoprotein is chosen among species classified in the vesiculovirus genus: Carajas virus (CJSV), Chandipura virus (CHPV), Cocal virus (COCV), Isfahan virus (ISFV), Maraba virus (MARAV), Piry virus (PIRYV), Vesicular stomatitis Alagoas virus (VSAV), Vesicular stomatitis Indiana virus (VSIV) and Vesicular stomatitis New Jersey virus (VSNJV) and/or Grass carp rhabdovirus, BeAn 157575 virus (BeAn 157575), Boteke virus (BTKV), Calchaqui virus (CQIV), Eel virus American (EVA), Gray Lodge virus (GLOV), Jurona virus (JURV), Klamath virus (KLAV), Kwatta virus (KWAV), La Joya virus (LJV), Malpais Spring virus (MSPV), Mount Elgon bat virus (MEBV), Perinet virus (PERV), Pike fry rhabdovirus (PFRV), Porton virus (PORV), Radi virus (RADIV), Spring viremia of carp virus (SVCV), Tupaia virus (TUPV), Ulcerative disease rhabdovirus (UDRV) and Yug Bogdanovac virus (YBV). In particular, the VSV-G protein originating from a VSV is modified with respect to its native form, especially to improve pseudotyping. In particular, the envelope protein comprises domains or fragments originating from different envelope protein(s) of different viruses, especially of different genus of different species of VSV. In the case of VSV, the G protein comprises or consists of the transmembrane domain of the indiana VSV and the ectodomain of a strain of a different VSV serotype. In particular, the envelope protein(s) comprises the transmembrane domain of the indiana VSV and the ectodomain of the New-Jersey VSV. In another aspect, the retroviral vector of the present disclosure is pseudotyped with HA protein (influenza-hemaglutinin), RD114 protein, modified envelopes with inserted cell-specific ligands or with viral envelope proteins originated from a virus selected in one or several of the following orders or families: Arenaviridae, Flaviridae, Togaviridae, Coronaviridae, Orthomyxoviridae, Retroviridae and Mononegavirales including Paramyxoviridae, Rhabdoviridae or Filoviridae. Any virus envelope protein is suitable for pseudotyping, assuming that the pseudotyping is compatible with production and purification steps of lentiviral vector particles.

The structure and composition of the vector genome used to prepare the retroviral vectors of the present disclosure are in accordance with those described in the art. Especially, minimum retroviral gene delivery vectors can be prepared from a vector genome, which only contains, apart from the heterologous polynucleotide(s) of interest (i.e. the transgene(s)), the sequences of the retroviral genome which are non-coding regions of said genome, necessary to provide recognition signals for DNA or RNA synthesis and processing. Hence, a vector genome may be a replacement vector in which all the viral coding sequences between the 2 long terminal repeats (LTRs) have been replaced by the polynucleotide(s) of interest (i.e. the transgene). In particular the retroviral vector genome also comprises in addition, a polynucleotide consisting in the DNA flap. The DNA flap (defined in Zennou V. et al 2000, Cell vol 101, 173-185 or in WO 99/55892 and WO 01/27304), is a structure which is central in the genome of some lentiviruses especially in HIV retroviruses, where it gives rise to a 3-stranded DNA structure normally synthesized during especially HIV reverse transcription and which acts as a cis-determinant of HIV genome nuclear import. The DNA flap enables a central strand displacement event controlled in cis by the central polypurine tract (cPPT) and the central termination sequence (CTS) during reverse transcription. When inserted in retroviral derived vectors, the polynucleotide enabling the DNA flap to be produced during retro-transcription, stimulates gene transfer efficiency and complements the level of nuclear import to wild-type levels (Arhel N. et al, Retrovirology 2006, 3:38, 26 June 2006, Wild- type and central DNA flap defective HIV-1 retroviral vector genomes: intracellular visualization at ultra structural resolution levels). In particular, the DNA flap is inserted immediately upstream of the transgene(s), advantageously to have a central position in the vector genome. A DNA flap suitable for the disclosure may be obtained from a retrovirus, especially from a lentivirus, or from a retrovirus-like organism such as retrotransposon, either prepared synthetically (chemical synthesis) or by amplification of the DNA flap from any retrovirus especially from a lentivirus nucleic acid such as by Polymerase chain reaction (PCR). The DNA flap may be obtained from a retrovirus, especially a lentivirus, especially from a human retrovirus or lentivirus and in particular a HIV retrovirus, or from the CAEV (Caprine Arthritis Encephalitis Virus) virus, the EIAV (Equine Infectious Anaemia Virus) virus, the VISNA virus, the SIV (Simian Immunodeficiency Virus) virus or the FIV (Feline Immunodeficiency Virus) virus. In particular, the DNA flap is obtained from an HIV retrovirus, for example HIV-1 or HIV-2 virus including any isolate of these two types. It is noteworthy that the DNA flap is used as a DNA fragment isolated from its natural (viral genome) nucleotide context i.e., out of the context of the pol gene in which it is naturally contained in the lentivirus genome. Therefore, the DNA flap is used, in the present disclosure, deleted from the unnecessary 5' and 3' parts of the pol gene and is recombined with sequences of different origin. The DNA flap may be either prepared synthetically (chemical synthesis) or by amplification of the DNA providing the DNA flap from the appropriate source as defined above such as by Polymerase chain reaction (PCR). A particular appropriate polynucleotide comprising the structure providing the DNA flap is a 178-base pair polymerase chain reaction (PCR) fragment encompassing the cPPT and CTS regions of the HIV-1 DNA

The retroviral vector genome may also comprise regulatory signals for transcription and expression of non retroviral origin, such as a promoter and/or an enhancer. Examples of promoters that can be used in immune response elicitation are CMV also referred to as CMVie (CMV immediate early), EF1α promoter, CGA promoter, CD11c promoter and house keeping gene promoters such as PGK promoter, ubiquitin promoter, actin promoter, histone promoter, alpha-tubulin promoter, beta-tubulin promoter, superoxide dismutase 1 (SOD-1) promoter, dihydrofolate reductase (DHFR) promoter, hypoxanthine phosphorybosyltransferase (HPRT) promoter, adenosine deaminase promoter, thymidylate synthetase promoter, dihydrofolate reductase PI promoter, glucose-6-phosphate sehydrogenase promoter or nucleolin promoter.

In particular, the transgene is under the control of regulatory signals for transcription and expression.

In particular, the retroviral vector genome comprises all the elements necessary for the nucleic import and the correct expression of the polynucleotide of interest (i.e. the transgene). As examples of elements that can be inserted in the retroviral genome of the retroviral vector of the present disclosure are at least one (preferably two) long terminal repeats (LTR), such as a LTR5' and a LTR3', a psi sequence involved in the retroviral genome encapsidation, and optionally at least one DNA flap comprising a cPPT and a CTS domains.

In particular of the present disclosure, the LTR, preferably the LTR3', is deleted for the promoter and the enhancer of U3; this modification has been shown to increase substantially the transcription of the transgene inserted in the retroviral genome (WO01/27304).

In particular, the retroviral vector genome may also comprise elements selected among a splice donor site (SD), a splice acceptor site (SA) and/or a Rev-responsive element (RRE).

In particular, the retroviral vector genome is devoid of functional gag, pol and/or env retroviral genes. By "functional" it is meant a gene that is correctly transcribed, and/or correctly expressed. Thus, the retroviral vector genome of the present disclosure may contain at least one of the gag, pol and env genes that is either not transcribed or incompletely transcribed; the expression "incompletely transcribed" refers to the alteration in the transcripts gag, gag-pro or gag-pro-pol, one of these or several of these being not transcribed. In particular, the retroviral genome is devoid of gag, pol and/or env retroviral genes.

In particular the retroviral vector genome is also devoid of the coding sequences for Vif-, Vpr-, Vpu- and Nef-accessory genes (for HIV-1 retroviral vectors), or of their complete or functional genes.

Typically, the retroviral vector of the present disclosure is non replicative i.e., the vector and retroviral vector genome are not able to form new particles budding from the infected host cell. This may be achieved by the absence in the retroviral genome of the gag, pol or env genes, as indicated in the above paragraph; this can also be achieved by deleting other viral coding sequence(s) and/or cis-acting genetic elements needed for particles formation.

In particular, the retroviral vector of the present disclosure comprises (i) a recombinant genome comprising, between the LTR sequences 5 'and 3' retroviral, a psi sequence retroviral packaging, a nuclear export element RNA, at least one transgene and, optionally, a promoter and / or a sequence favoring the nuclear import of the RNA, and (ii) a chimeric integrase protein of the present disclosure. In particular, the recombinant genome comprises for the sequence 5 'LTR-psi-RRE-cPPT-CTS-transgene(s)-3' LTR. In particular, the recombinant genome comprises the sequence 5 'LTR-psi-RRE-cPPT-CTS-promoter-transgene(s)-3' LTR. In particular, the recombinant genome comprises the sequence 5 'LTR-psi-RRE-cPPT-transgene(s)-CTS-3' LTR.

As used herein, the term "transgene" or "polynucleotide of interest" refers to any nucleic acid that shall be expressed in a mammal cell. Typically the nucleic acid is a coding or non coding nucleic acid. It can be a non-coding sequence such as for example a recognition sequence of an enzyme (site specific integration, site with a particular affinity for a protein, etc.).. This is preferably a sequence encoding a given polypeptide or RNA active as such. It may include a cDNA, a gDNA, a synthetic DNA, an RNA, for example, a siRNA, a ribozyme, etc.. or a combination thereof. Typically, the transgene is a DNA comprising a sequence encoding the desired expression product. The transgene may also include one or more regions of transcription termination, typically a polyadenylation signal.

In particular, the transgene may be selected from a nucleic acid catalyst (interfering, antisense, ribozyme), a nucleic acid suicide (eg, encoding a toxin) or a nucleic acid encoding a biologically active peptide, such as a growth factor, a trophic factor, one anti-angiogenic factor, a hormone, a cytokine, an antibody, a receptor, a differentiation factor, a colony stimulating factor, an anticancer agent, an enzyme, a neurotransmitter or its precursor, etc. In particular, the transgene encodes eg trophic factors include: RdCVF, CNTF, NGF, NT3, NT4, FGF, PDGF, GDNF, etc.., Or for anti-angiogenic factors or enzymes restaurant deficient metabolic activity or providing a particular metabolic function, for example: TH, AADC, GTPC, β-glucuronidase, etc..

In particular, the transgene encodes, for example, RNA interference (RNAi) to inhibit specifically the expression of mutated proteins involved in a disease or a dominant genetic disease caused by a gain of function, such as a neurodegenerative disease such as mutated SOD (Amyotrophic Lateral Sclerosis), protein APP, tau, presenilin, or BACE (Alzheimer's disease), the α-synuclein (Parkinson's disease) or Huntingtin (Huntington disease).

In certain circumstances, the transgene encodes for a site-specific endonuclease that provides for site-specific knock-down of gene function. In particular, the transgene encodes for a zinc-finger protein (ZFP) that binds to target site in a region of interest (e.g., a disease associated gene, a highly expressed gene, an albumin gene or other or safe harbor gene) in a genome, wherein the ZFP comprises one or more engineered zinc-finger binding domains. In particular, the ZFP is a zinc-finger nuclease (ZFN) that cleaves a target genomic region of interest, wherein the ZFN comprises one or more engineered zinc-finger binding domains and a nuclease cleavage domain or cleavage half-domain. Cleavage domains and cleavage half domains can be obtained, for example, from various restriction endonucleases and/or homing endonucleases. In particular, the cleavage half-domains are derived from a Type IIS restriction endonuclease (e.g., Fok I). In particular, the zinc finger domain recognizes a target site in a disease associated or safe harbor gene such as albumin. In particular, the transgene encodes for a TALE protein (Transcription activator like) that binds to target site in a region of interest (e.g., a highly expressed gene, a disease associated gene or a safe harbor gene) in a genome, wherein the TALE comprises one or more engineered TALE binding domains. In particular, the TALE is a nuclease (TALEN) that cleaves a target genomic region of interest, wherein the TALEN comprises one or more engineered TALE DNA binding domains and a nuclease cleavage domain or cleavage half-domain. Cleavage domains and cleavage half domains can be obtained, for example, from various restriction endonucleases and/or homing endonucleases. In particular, the cleavage half-domains are derived from a Type IIS restriction endonuclease (e.g., Fok I). In particular, the TALE DNA binding domain recognizes a target site in a highly expressed, disease associated, or safe harbor gene. In particular, the transgene encodes for a CRISPR/Cas system that binds to target site in a region of interest (e.g., a highly expressed gene, a disease associated gene or a safe harbor gene) in a genome, wherein the CRISPR/Cas system comprises a CRIPSR/Cas nuclease and an engineered crRNA/tracrRNA (or single guide RNA). In particular, the CRISPR/Cas system recognizes a target site in a highly expressed, disease associated, or safe harbor gene. The ZFN, TALEN, and/or CRISPR/Cas system as described herein may bind to and/or cleave the region of interest in a coding or non-coding region within or adjacent to the gene, such as, for example, a leader sequence, trailer sequence or intron, or within a non-transcribed region, either upstream or downstream of the coding region. In particular, the ZFN, TALEN, and/or CRISPR/Cas system binds to and/or cleaves a highly expressed gene, for example a globin gene in red blood cells (RBCS). See, e.g., U.S. Application No. 61/670,451, titled "Methods and Compositions for Delivery of Biologies," filed Jul. 11, 2012. In particular, the ZFN, TALEN, and/or CRISPR/Cas system binds to and/or cleaves a safe-harbor gene, for example a CCR5 gene, a PPP1R12C (also known as AAV SI) gene, albumin, HPRT or a Rosa gene. See, e.g., U.S. Patent Publication Nos. 20080299580; 20080159996 and 201000218264 and U.S. Application Nos. 61/537,349, 61/560,506, 13/660,821 and U.S. Application No. 61/670,451 titled "Methods and Compositions for Regulation of Transgene Expression" filed Jul. 11, 2012. In addition, to aid in selection, the HPRT locus may be used (see U.S. patent application Ser. Nos. 13/660,821 and 13/660,843). In particular, the ZFN, TALEN, and/or CRISPR/Cas system may bind to and/or cleave a disease associated gene (e.g. the gene encoding lysosomal hydrolase α-galactosidase A (AGA), related to Fabry's Disease).

In particular, the transgene encodes for an antigenic polypeptide. A polypeptide is said antigenic when its sequence contains at least one peptide (epitope) able to elicit an immune response when put in contact with antigen presenting cells (APC). Typically, the antigenic polypeptide comprises at least one B epitope, capable of eliciting a humoral immune response, particularly a protective humoral response, or a T-epitope capable of eliciting a cellular immune response. In particular, at least one polypeptide is encoded by a nucleotide sequence originating from the genome of a pathogen, such as a virus, especially a retrovirus, lentivirus, flavivirus or coronavirus, of a bacterium or of a parasite. In particular, the antigenic polypeptide of the present disclosure comprises or consists in surface antigens, such as viral envelope or other membrane proteins, and fragments thereof, for example envelope from AIDS viruses, including HIV-1 or HIV-2, or for example envelope from the Yellow Fever Virus, the West Nile Virus, the Dengue virus (DV), the Japanese encephalitis virus (JEV) or the SARS-associated coronavirus. Other interesting viral polypeptides are from the capsid of HIV. Alternatively, the antigenic polypeptide is derived from a tumoral antigen or a tumoral epitope. Particular polypeptides (or part thereof) are those expressed on the cell surface of tumoral cells. These polypeptides (or part thereof) may originate from the cell (self peptide) either in a wild type or mutated form; they also may originate from a virus that transforms a normal cell in tumor cell (tumor virus). Examples of such viruses, etiologic agents for human cancer, are the Human Papilloma Virus (HPV) causing cervical cancer, the Epstein-Barr Virus causing lymphoma through the EBV-induced membrane antigen (EBMA), HTLV-1 causing Acute T cell leukaemia (ACT) through the HTLV-1 tax protein, the human herpes virus type 8 (HHV8), the hepatitis B virus (HBV) and the hepatitis C virus (HCV).

The transgene is typically placed under the control of a transcriptional promoter, which may be homologous to the transgene-or heterologous promoter such as a cellular, viral, synthetic, chimeric, etc.. The promoter used may be constitutive or regulated, weak or strong, tissue-specific or ubiquitous dependent RNA polymerase 2 or 3, etc.. It typically uses a viral promoter such as CMV, RSV LTR, TK, etc.. or preferably a cellular promoter such as PGK, Rho, EF1α, etc.. Of tissue-specific promoters can be used. It may be, for example promoters ENO, GFAP, NSE, a promoter of RNA polymerase III promoter such as U6 or HI, possibly modified, etc.. The promoter used to drive expression of the transgene can be for example a viral promoter selected from the gene promoter CMV, RSV LTR or TK.

The retroviral vectors of the present disclosure can be produced by any well-known method in the art including by transfection (s) transient (s), in stable cell lines and / or by means of helper virus. Typically, the retroviral vector of the present disclosure is obtainable by a transcomplementation system (vector/packaging system) by transfecting in vitro a permissive cell (such as 293T cells) with a plasmid containing the retroviral vector genome of the present disclosure, and at least one other plasmid providing, in trans, the gag, pol and env sequences encoding the polypeptides GAG, POL and the envelope protein(s), or for a portion of these polypeptides sufficient to enable formation of retroviral particles. As an example, permissive cells are transfected with a) transcomplementation plasmid, lacking packaging signal psi and comprising a sequence retroviral gag and pol sequence encoding a chimeric integrase protein of the present disclosure, the plasmid is optionally deleted of accessory genes vif, nef, vpu and / or vpr, b) a second plasmid (envelope expression plasmid or pseudotyping env plasmid) comprising a gene encoding an envelope protein(s) (such as VSV-G) and c) a plasmid vector comprising a recombinant genome retroviral, optionally deleted from the promoter region of the 3 'LTR or U3 enhancer sequence of the 3' LTR, including, between the LTR sequences 5 'and 3' retroviral, a psi encapsidation sequence, a nuclear export element (preferably RRE element of HIV or other retroviruses equivalent), the transgene and optionally a promoter and / or a nuclear import sequence (cPPT sequence eg CTS) of the RNA. Advantageously, the three plasmids used do not contain homologous sequence sufficient for recombination. Nucleic acids encoding gag, pol and env cDNA can be advantageously prepared according to conventional techniques, from viral gene sequences available in the prior art and databases. The transcomplementation plasmid provides a nucleic acid encoding the proteins retroviral gag and pol. These proteins are derived from a lentivirus, and most preferably, from HIV-1. The plasmid is devoid of encapsidation sequence, sequence coding for an envelope, accessory genes, and advantageously also lacks retroviral LTRs. Therefore, the sequences coding for gag and pol proteins are advantageously placed under the control of a heterologous promoter, eg cellular, viral, etc.., which can be constitutive or regulated, weak or strong. It is preferably a plasmid containing a sequence transcomplémentant Δpsi-CMV-gag-pol-PolyA. This plasmid allows the expression of all the proteins necessary for the formation of empty virions, except the envelope glycoproteins. The plasmid transcomplementation may advantageously comprise the TAT and REV genes. Plasmid transcomplementation is advantageously devoid of vif, vpr, vpu and / or nef accessory genes. It is understood that the gag and pol genes and genes TAT and REV can also be carried by different plasmids, possibly separated. In this case, several plasmids are used transcomplementation, each encoding one or more of said proteins. The promoters used in the plasmid transcomplementation, the envelope plasmid and the plasmid vector respectively to promote the expression of gag and pol of the coat protein, the mRNA of the vector genome and the transgene are promoters identical or different, chosen advantageously from ubiquitous promoters or specific, for example, from viral promoters CMV, TK, RSV LTR promoter and the RNA polymerase III promoter such as U6 or HI or promoters of helper viruses encoding env, gag and pol (i.e. adenoviral, baculoviral, herpes viruses). For the production of the retroviral vector of the present disclosure, the plasmids described above can be introduced into competent cells and viruses produced are harvested. The cells used may be any cell competent, particularly eukaryotic cells, in particular mammalian, eg human or animal. They can be somatic or embryonic stem or differentiated. Typically the cells include 293T cells, fibroblast cells, hepatocytes, muscle cells (skeletal, cardiac, smooth, blood vessel, etc.)., nerve cells (neurons, glial cells, astrocytes) of epithelial cells, renal, ocular etc.. It may also include, insect, plant cells, yeast, or prokaryotic cells. It can also be cells transformed by the SV40 T antigen. The genes gag, pol and env encoded in plasmids or helper viruses can be introduced into cells by any method known in the art, suitable for cell type considered. Usually, the cells and the vector system are contacted in a suitable device (plate, dish, tube, pouch, etc...), for a period of time sufficient to allow the transfer of the vector system or the plasmid in the cells. Typically, the vector system or the plasmid is introduced into the cells by calcium phosphate precipitation, electroporation, transduction or by using one of transfection-facilitating compounds, such as lipids, polymers, liposomes and peptides, etc.. The calcium phosphate precipitation is preferred. The cells are cultured in any suitable medium such as RPMI, DMEM, a specific medium to a culture in the absence of fetal calf serum, etc. Once transfected the retroviral vectors of the present disclosure may be purified from the supernatant of the cells. Purification of the retroviral vector to enhance the concentration can be accomplished by any suitable method, such as by density gradient purification (e.g., cesium chloride (CsCl)) or by chromatography techniques (e.g., column or batch chromatography). For example, the vector of the present disclosure can be subjected to two or three CsCl density gradient purification steps. The vector, is desirably purified from cells infected using a method that comprises lysing cells infected with adenovirus, applying the lysate to a chromatography resin, eluting the adenovirus from the chromatography resin, and collecting a fraction containing the retroviral vector of the present disclosure.

The retroviral vector of the present disclosure is particularly suitable for driving the targeted integration into the genome of the infected cell. Thus a further object of the present disclosure relates to a method for expressing a transgene into the genome of a plurality of cells comprising infecting the plurality of cells with the retroviral vector of the present disclosure. The retroviral vector of the present disclosure thus can be used for expressing the transgene in a mammal cell of interest, more particularly in cells that do not divide or the transient expression of the transgene in division cells that are refractory to other methods of transfection or transduction. The retroviral vectors of the present disclosure are able to transduce various cell types such as, for example, liver cells (e.g. hepatocytes), muscle cells, brain cells, kidney cells, retinal cells, and hematopoietic cells. In particular, the target cells of the present disclosure are "non-dividing" cells. These cells include cells such as neuronal cells that do not normally divide. However, it is not intended that the present disclosure be limited to non-dividing cells (including, but not limited to muscle cells, white blood cells, spleen cells, liver cells, eye cells, epithelial cells, etc.). Possible applications of retroviral vectors of the present disclosure are of several types and include gene therapy, ie, the gene transfer in any mammal cell, in particular in human cells. It may be dividing cells or quiescent cells, cells belonging to the central organs or peripheral organs such as the liver, pancreas, muscle, heart, etc.. This is preferably a gene transfer into quiescent cells (which do not divide), Gene therapy may allow the expression of proteins, eg neurotrophic factors, enzymes, transcription factors, receptors, etc.. It also enables to implement a strategy "oligonucleotide" (interfering RNA or antisense, ribozymes, etc..) cell therapy, ie, the expression of differentiation factors in progenitor cells to guide cell fate to a selected before transplantation or ex vivo transduction of cells to express an interest factor, followed by transplantation of the said cells.

Retroviral vectors of the present disclosure may also particularly suitable for research purposes.

Retroviral vectors of the present disclosure are also particularly suitable for preparing genetically modified organism. By "genetically modified" is meant a gene that is altered from its native state (e.g. by insertion mutation, deletion mutation, nucleic acid sequence mutation, or other mutation), or that a gene product is altered from its natural state (e.g. by delivery of a transgene that works in trans on a gene's encoded mRNA or protein, such as delivery of inhibitory RNA or delivery of a dominant negative transgene). Typically said genetically-modified organism may be any organism of plant or animal origin, which is used as food or feed, for producing crops or manufacture material, or for producing transgenic animals for food or feed, or stock breeding.

Retroviral vectors of the present disclosure may also be particularly suitable for the production of vaccines or for eliciting a vaccine response in a subject in need thereof.

The retroviral vector of the present disclosure may also be used as a medicament. The retroviral vector of the present disclosure may be particularly suitable for treating a disease in a subject.

In particular, the retroviral vector of the present disclosure can be used to treat a cancer. Non-limiting examples of cancers that can be treated of the present disclosure include breast cancer, prostate cancer, lymphoma, skin cancer, pancreatic cancer, colon cancer, melanoma, malignant melanoma, ovarian cancer, brain cancer, primary brain carcinoma, head-neck cancer, glioma, glioblastoma, liver cancer, bladder cancer, non-small cell lung cancer, head or neck carcinoma, breast carcinoma, ovarian carcinoma, lung carcinoma, small-cell lung carcinoma, Wilms' tumor, cervical carcinoma, testicular carcinoma, bladder carcinoma, pancreatic carcinoma, stomach carcinoma, colon carcinoma, prostatic carcinoma, genitourinary carcinoma, thyroid carcinoma, esophageal carcinoma, myeloma, multiple myeloma, adrenal carcinoma, renal cell carcinoma, endometrial carcinoma, adrenal cortex carcinoma, malignant pancreatic insulinoma, malignant carcinoid carcinoma, choriocarcinoma, mycosis fungoides, malignant hypercalcemia, cervical hyperplasia, leukemia, acute lymphocytic leukemia, chronic lymphocytic leukemia, acute myelogenous leukemia, chronic myelogenous leukemia, chronic granulocytic leukemia, acute granulocytic leukemia, hairy cell leukemia, neuroblastoma, rhabdomyosarcoma, Kaposi's sarcoma, polycythemia vera, essential thrombocytosis, Hodgkin's disease, non-Hodgkin's lymphoma, soft-tissue sarcoma, mesothelioma, osteogenic sarcoma, primary macroglobulinemia, and retinoblastoma, and the like.

In particular, the retroviral vector of the present disclosure can be used to treat an autoimmune disorder including, but not limited to, a disorder selected from the group consisting of Achlorhydra Autoimmune Active Chronic Hepatitis, Acute Disseminated Encephalomyelitis, Acute hemorrhagic leukoencephalitis, Addison's Disease, gammaglobulinemia, Agammaglobulinemia, Alopecia areata, Amyotrophic Lateral Sclerosis, Ankylosing Spondylitis, Anti-GBM/TBM Nephritis, Antiphospholipid syndrome, Antisynthetase syndrome, Arthritis, Atopic allergy, Atopic Dermatitis, Aplastic Anemia, Autoimmune cardiomyopathy, Autoimmune hemolytic anemia, Autoimmune hepatitis, Autoimmune inner ear disease, Autoimmune lymphoproliferative syndrome, Autoimmune peripheral neuropathy, Autoimmune pancreatitis, Autoimmune polyendocrine syndrome Types I, II, & III, Autoimmune progesterone dermatitis, Autoimmune thrombocytopenic purpura, Autoimmune uveitis, Balo disease/Balo concentric sclerosis, Bechets Syndrome, Berger's disease, Bickerstaff s encephalitis, Blau syndrome, Bullous Pemphigoid, Castleman's disease, Chronic Fatigue Immune Dysfunction Syndrome, chronic inflammatory demyelinating polyneuropathy, Chronic recurrent multifocal ostomyelitis, Churg-Strauss syndrome, Cicatricial Pemphigoid, Coeliac Disease, Cogan syndrome, Cold agglutinin disease, Complement component 2 deficiency, Cranial arteritis, CREST syndrome, Crohns Disease, Cushing's Syndrome, Cutaneous leukocytoclastic angiitis, Dego's disease, Dermatitis herpetiformis, Dermatomyositis, Diabetes mellitus type 1, Diffuse cutaneous systemic sclerosis, Dressler's syndrome, Discoid lupus er thematosus, eczema, Enthesitis-related arthritis, Eosinophilic fasciitis, Epidermolysis bullosa acquisita, Erythema nodosum, Essential mixed cryoglobulinemia, Evan's syndrome, Fibrodysplasia ossificans progressiva., Fibromyositis, Fibrosing aveolitis, Gastritis, Gastrointestinal pemphigoid. Giant cell arteritis, Goodpasture's syndrome, Graves' disease. Guillain-Barre syndrome (GBS), Hashimoto's encephalitis, Hashimoto's thyroiditis, Hemolytic anaemia, Henoc -Schonlein purpura, Herpes gestationis, Hughes syndrome (or Antiphospholipid syndrome). Hypogammaglobulinemia, Idiopathic Inflammatory Demyelinating Diseases, Idiopathic pulmonary fibrosis, Idiopathic thrombocytopenic purpura, IgA nephropathy (or Bergefs disease), Inclusion body myositis, ory demyelinating polyneuopathy, Juvenile idiopathic arthritis, Juvenile rheumatoid arthritis, Lambert-Eaton myasthenic syndrome, Leukocytoclastic vasculitis, Lichen planus, Lichen sclerosus, Linear IgA disease (LAD), Lou Gehrig's Disease, Lupoid hepatitis, Lupus erythematosus, Majeed syndrome, Meniere's disease, Microscopic polyangiitis, Miller-Fisher syndrome, Mixed Connective Tissue Disease, Mucha-Habermann disease, Muckle-Wells syndrome, Multiple Myeloma, Myasthenia gravis, Myositis, Narcolepsy, Neuromyelitis optica (also Devic's Disease), Occular cicatricial pemphigoid, Ord thyroiditis, Palindromic rheumatism, PANDAS (Pediatric Autoimmune Neuropsychiatric Disorders Associated with Streptococcus), Paraneoplastic cerebellar degeneration, Paraneoplastic cerebellar degeneration, Parry Romberg syndrome, Parsonnage-Turner syndrome, Pars planitis, Pemphigus, Pemphigus vulgaris, Pernicious anaemia, Perivenous encephalomyelitis, POEMS syndrome, Polyarteritis nodosa, Polymyalgia rheumatica, Polymyositis, Primary biliary cirrhosis, psoriasis, psoriatic arthritis, Pyoderma gangrenosum, pure red cell aplasia, Rasmussen's encephalitis, Raynaud phenomenon, Relapsing polychondritis, Reiter's syndrome, Retroperitoneal fibrosis, Rheumatoid arthritis, Rheumatoid fever, Schmidt syndrome, Schnitzler syndrome, Scleritis, Sjogren's syndrome, Spondyloarthropathy, sticky blood syndrome, Still's Disease, Subacute bacterial endocarditis (SBE), Susac's syndrome, Sweet syndrome, Sydenham Chorea, Sympathetic ophthalmia, Takayasu's arteritis, Temporal arteritis, Tolosa-Hunt syndrome, Transverse Myelitis, Ulcerative Colitis, Undifferentiated connective tissue disease, Undifferentiated spondyloarthropathy, vasculitis, Wegener's granulomatosis, Wilson's syndrome, and Wiskott-Aldrich syndrome.

In particular, the retroviral vector of the present disclosure can be used to treat an ocular disorder that includes, but is not limited to, a disorder selected from the group consisting of glaucoma including Open Angle Glaucoma (e.g., Primary Open Angle Glaucoma, Pigmentary Glaucoma, and Exfoliative Glaucoma, Low Tension Glaucoma), Angle Closure Glaucoma (also known clinically as closed angle glaucoma, narrow angle glaucoma, pupillary block glaucoma, and ciliary block glaucoma) (e.g., Acute Angle Closure Glaucoma and Chronic Angle Closure Glaucoma), Aniridic Glaucoma, Congenital Glaucoma, Juvenile Glaucoma, Lens-Induced Glaucoma, Neovascular Glaucoma (e.g., using vectors composed of Vascular Endothelial Growth Factor (VEGF) decoy, Pigment Derived Growth Factor (PDGF), Endostatin, Angiostatin, or Angiopoetin-1), Post-Traumatic Glaucoma, Steroid-Induced Glaucoma, Sturge- Weber Syndrome Glaucoma, and Uveitis-Induced Glaucoma, diabetic retinopathy (e.g., using vectors composed of VEGF decoy, PDGF, Endostatin, Angiostatin, or Angiopoetin-1), macular degeneration (e.g. vectors composed of VEGF decoy, PDGF, Endostatin, Angiostatin, Angiopoetin-1, ATP Binding Casette Subfamily A Member 4), macular degeneration (e.g., using vectors composed of VEGF decoy, PDGF, Endostatin, Angiostatin, Angiopoetin-1, ATP Binding Casette Subfamily A Member 4), choroidal neovascularization, (e.g., using vectors composed of VEGF decoy, PDGF, Endostatin, Angiostatin, or Angiopoetin-1), vascular leak, and/or retinal edema, bacterial conjunctivitis, fungal conjunctivitis, viral conjunctivitis, uveitis, keratic precipitates, macular edema (e.g., using vectors composed of VEGF decoy, PDGF, Endostatin, Angiostatin, or Angiopoetin-1), inflammation response after intra-ocular lens implantation, uveitis syndromes (for example, chronic iridocyclitis or chronic endophthalmitis), retinal vasculitis (for example, as seen in rheumatoid arthritis, juvenile rheumatoid arthritis, systemic lupus erythematosus, progressive systemic sclerosis, polyarteritis nodosa, Wegener's granulomatosis, termporal arteritis, Adamantiades Bechcet disease, Sjorgen's, relapsing polychondritis and HLA-B27 associated spondylitis), sarcoidosis, Eales disease, acute retinal necrosis, Vogt Koyanaki Harada syndrome, occular toxoplasmosis, radiation retinopathy, proliferative vitreoretinopathy, endophthalmitis, ocular glaucomas (for example, inflammatory glaucomas), optic neuritis, ischemic optic neuropathy (e.g. vectors composed of Allotopic NADH dehydrogenase Unit 4), thyroid associated orbitopathy, orbital pseudotumor, pigment dispersion syndrome (pigmentary glaucoma), scleritis, episcleritis choroidopathies (for example, "White-dot" syndromes including, but not limited to, acute multifocal posterior placoid), retinopathies (for example, cystoid macular edema, central serous choroidopathy and presumed ocular histoplasmosis syndrome (e.g., vectors composed of Glial Cell Derived Neurotropic Factor, Peripherin-2)), retinal vascular disease (for example, diabetic retinopathy, Coat's disease and retinal arterial macroaneurysm), retinal artery occlusions, retinal vein occlusions, retinopathy of prematurity, retinitis pigmentosa (e.g. vectors composed of Retinal Pigment Specific 65kDa protein), familial exudative vitreoretinopathy (FEVR), idiopathic polypoidal choroidal vasculopathy, epiretinal macular membranes and cataracts.

In particular, the retroviral vector of the present disclosure can be used to treat a blood disorder that includes, but is not limited to, a blood disorder selected from the group consisting of anemia, bleeding and clotting disorders (e.g., disseminated intravascular coagulation (DiC), hemophilia, Henoch-Schonlien Purpura, hereditary hemorrhagic telangiectasia, thrombocytopenia (ITP, TTP), thrombophilia, Von Willebrand's disease), leukemias (e.g., acute lymphocytic leukemia, acute myelocytic leukemia, chronic lymphocytic leukemia, chronic myelocytic leukemia), lymphomas (e.g., Hodgkin lymphoma, non-Hodgkin lymphoma), myeloproliferative disorders (e.g., myelofibrosis, Polycythemia Vera, thrombocythemia), plasma cell disorders (e.g., macroglobulinemia, monoclonal gammopathies of undetermined significance, multiple lyeloma), spleen disorders, white blood cell disorders (e.g., basophilic disorder, eosinophilic disorder, lymphocytopenia, monocyte disorders, neutropenia, neutrophillic leukocytosis), thrombosis, deep vein thrombosis (DVT), hemochromatosis, menorrhagia, sickle cell disease, and thalassemia.

In particular, the retroviral vector of the present disclosure can be used to treat a neurological disorder that includes, but is not limited to, a neurological disorders selected from the group consisting of Gaucher disease, Parkinson's disease, Alzheimer's disease, amyotrophic lateral sclerosis (ALS), multiple sclerosis (MS), Huntington's disease, Fredrich's ataxia, Mild Cognitive Impairment, Cerebral Amyloid Angiopathy, Parkinsonism Disease, Lewy Body Disease, Frontotemporal Dementia (FTD) Multiple System Atrophy (MSA), Progressive Supranuclear Palsy, and movement disorders (including ataxia, cerebral palsy, choreoathetosis, dystonia, Tourette's syndrome, kernicteras) and tremor disorders, and leukodystrophies (including adrenoleukodystrophy, metachromatic leukodystrophy, Canavan disease, Alexander disease, Pelizaeus-Merzbacher disease), neuronal ceroid lipofucsinoses, ataxia telangectasia, Rett Syndrome, alpha.-synucleinopathy (e.g., Lewy Body Disease, Multiple System Atrophy, Hallervorden-Spatz disease, or Frontotemporal Dementia), Niemann- Pick Type C disease (NPCD), spinocerebellar ataxia Type 1 , Type 2, and Type 3, and dentatorubral pallidoluysian atrophy (DRLPA).

In particular, the retroviral vector of the present disclosure can be used to treat a lung disorder that includes, but is not limited to, a lung disorder selected from the group consisting of asthma, atelectasis, bronchitis, COPD (chronic obstructive pulmonary disease), emphysema, Lung cancer, mesothelioma, pneumonia, asbestosis, Aspergilloma, Aspergillosis, Aspergillosis - acute invasive, bronchiectasis, bronchiolitis obliterans organizing pneumonia (BOOP), eosinophilic pneumonia, necrotizing pneumonia, ral effusion, pneumoconiosis, pneumothorax, pulmonary actinomycosis, monary alveolar proteinosis, pulmonary anthrax, pulmonary arteriovenous malformation, pulmonary fibrosis, pulmonary embolus, pulmonary histiocytosis X (eosinophilic granuloma), pulmonary hypertension, pulmonary edema, pulmonary hemorrhage, pulmonary nocardiosis, pulmonary tuberculosis, pulmonary veno-occlusive disease, rheumatoid lung disease, sarcoidosis, radiation fibrosis, hypersensitivity pneumonitis, acute respiratory distress syndrome (ARDS), infant respiratory distress syndrome, idiopathic pulmonary fibrosis, idiopathic interstitial pneumonia, lymphangioleiomyomatosis, pulmonary Langerhans' cell histiocytosis, pulmonary alveolar proteinosis, sinusitis, tonsillitis, otitis media, pharyngitis, laryngitis, Pulmonary hamartoma, pulmonary sequestration, congenital cystic adenomatoid malformation (CCAM), and cystic fibrosis.

In particular, the retroviral vector of the present disclosure can be used to treat an infectious disease in a human that includes, but is not limited to, an infectious disease selected from the group consisting of fungal diseases such as dermatophytosis (e.g., trichophytosis, ringworm or tinea infections), athletes foot, paronychia, pityriasis versicolor, erythrasma, intertrigo, fungal diaper rash, Candida vulvitis, Candida balanitis, otitis externa, candidiasis (cutaneous and mucocutaneous), chronic mucocandidiasis (e.g. thrush and vaginal candidiasis), cryptococcosis, geotrichosis, trichosporosis, aspergillosis, penicilliosis, fusariosis, zygomycosis, sporotrichosis, chromomycosis, coccidioidomycosis, histoplasmosis, blastomycosis, paracoccidioidomycosis, pseudallescheriosis, mycetoma, mycotic keratitis, otomycosis, pneumocystosis, and fungemia, Acinetobacter infections, Actinomycosis, African sleeping sickness, AIDS (Acquired immune deficiency syndrome), Amebiasis, Anaplasmosis, Anthrax, Arcanobacterium haemolyticum infection, Argentine hemorrhagic fever, Ascariasis, Aspergillosis, atrovirus infection, Babesiosis, Bacillus cereus infection, Bacterial pneumonia, Bacterial vaginosis (BV), Bacteroides infection, Balantidiasis, Baylisascaris infection, BK virus infection, Black piedra, Blastocystis hominis infection, Borrelia infection, Botulism (and Infant botulism), Brazilian hemorrhagic fever, Brucellosis, Burkholderia infection, Buruli ulcer, Calcivirus infection (Norovirus and Sapovirus), Candidiasis, Cat-scratch disease, Cellulitis, Chagas Disease (American trypanosomiasis), Chancroid, Chickenpox, Chlamydia, Cholera, Chromoblastomycosis, Clonorchiasis, Clostridium difficile, Coccidioidomycosis, Colorado tick fever (CTF), Common cold (Acute viral rhinopharyngitis; Acute coryza), Creutzfeldt-Jakob disease (CJD), Cryptococcosis, Cryptosporidiosis, ous larva migrans (CLM), Dengue fever, Dientamoebiasis, Diphtheria, Diphyllobothriasis, Diphyllobothriasis, Dracunculiasis, Ebola hemorrhagic fever, Echinococcosis, Ehrlichiosis, Enterobiasis (Pinworm infection), Enterococcus infection, Enterovirus infection, Epidemic typhus, Erythema infectiosum, Exanthem subitum, Fasciolopsiasis, Fasciolosis, Fatal familial insomnia (FFI), Filariasis, Fusobacterium infection, Gas gangrene (Clostridial myonecrosis), Geotrichosis, Gerstmann-Straussler-Scheinker syndrome (GSS), Giardiasis Glanders, Gnathostomiasis, Gonorrhea, Granuloma inguinale (Donovanosis), Group A streptococcal infection, Group B streptococcal infection, Haemophilus influenzae, Hand, foot and mouth disease (HFMD), Hantavirus Pulmonary Syndrome (HPS) Helicobacter pylori infection, ic-uremic syndrome (HUS), Hemorrhagic fever with renal syndrome (HFRS), Hepatitis A, B, C, D, E, Herpes simplex, Histoplasmosis, Hookworm infection, n bocavirus infection, Human ewingii ehrlichiosis, Human granulocytic anaplasmosis (HGA), Human granulocytic anaplasmosis (HGA), Human monocytic ehrlichiosis, Human papillomavirus (HPV) infection, Human parainfluenza virus infection, Hymenolepiasis, Epstein-Barr Virus Infectious Mononucleosis (Mono), Influenza (flu), Isosporiasis, Kawasaki disease, Keratitis, Kingella kingae infection, Kuru, Lassa fever, Legionellosis (Legionnaires' disease), Legionellosis (Pontiac fever), Leishmaniasis, Leprosy, Leptospirosis, Listeriosis, Lyme disease (Lyme borreliosis), Lymphatic filariasis (Elephantiasis), Lymphocytic choriomeningitis, Malaria, Marburg hemorrhagic fever (MHF), Measles, Melioidosis (Whitmore's disease), Meningitis, Meningococcal disease, Metagonimiasis, Microsporidiosis, Molluscum contagiosum (MC), Mumps, Murine typhus (Endemic typhus), Mycoplasma pneumonia, Mycetoma, Myiasis, Neonatal conjunctivitis (Ophthalmia neonatorum), (New) Variant Creutzfeldt-Jakob disease (vCJD, nvCJD), Nocardiosis, Onchocerciasis (River blindness), Paracoccidioidomycosis (South American blastomycosis), Paragonimiasis, Pasteurellosis, Pediculosis capitis (Head lice), Pediculosis corporis (Body lice), Pediculosis pubis (Pubic lice, Crab lice), Pelvic inflammatory disease (PID), Pertussis (Whooping cough), Plague, Pneumococcal infection, Pneumocystis pneumonia (PCP), Pneumonia, Poliomyelitis, Poliomyelitis, Prevotella infection, mary amoebic meningoencephalitis (PAM), Progressive multifocal leukoencephalopathy, Psittacosis, Q fever, Rabies, Rat-bite fever, Respiratory syncytial virus infection, Rhinosporidiosis, inovirus infection, Rickettsial infection, Rickettsialpox, Rift Valley fever (RVF), Rocky mountain spotted fever (RMSF), Rotavirus infection, Rubella, Salmonellosis, SARS (Severe Acute Respiratory Syndrome), Scabies, Schistosomiasis, Sepsis, Shigellosis (Bacillary dysentery), Shingles (Herpes zoster), Smallpox (Variola), Sporotrichosis, Staphylococcal food poisoning, Staphylococcal infection, Strongyloidiasis, Syphilis, Taeniasis, tanus (Lockjaw), Tinea barbae (Barber's itch), Tinea capitis (Ringworm of the Scalp), Tinea corporis (Ringworm of the Body), Tinea cruris (Jock itch), Tinea manuum (Ringworm of the Hand), Tinea nigra, Tinea unguium (Onychomycosis), Tinea versicolor (Pityriasis versicolor). Toxocariasis (Visceral Larva Migrans (VLM)), Toxoplasmosis, Trichinellosis, Trichomoniasis, Trichuriasis (Whipworm infection), Tuberculosis, Tularemia, Ureaplasma real iicum infection, Venezuelan equine encephalitis, Venezuelan hemorrhagic fever, viral pneumonia. West Nile Fever, White plectra (Tinea blanca), Yersinia pseudotuberculosis infection, Yersiniosis, Yellow fever, and Zygomycosis.

Retroviral vectors of the present disclosure can be administered to a subject by any route. In particular the retroviral vector of the present disclosure is administered to the subject parenterally, preferably intravascularly (including intravenously). When administered parenterally, it is preferred that the vectors be given in a pharmaceutical vehicle suitable for injection such as a sterile aqueous solution or dispersion. Following administration, the subject is monitored to detect the expression of the transgene. Dose and duration of treatment is determined individually depending on the condition or disease to be treated. A wide variety of conditions or diseases can be treated based on the gene expression produced by administration of the gene of interest in the vector of the present disclosure. The dosage of vector delivered using the method of the present disclosure will vary depending on the desired response by the host and the vector used. Generally, it is expected that up to 100-200 µg of DNA or RNA can be administered in a single dosage, although a range of 0.5 mg/kg body weight to 50 mg/kg body weight will be suitable for most applications.

The disclosure will be further illustrated by the following figures and examples. However, these examples and figures should not be interpreted in any way as limiting the scope of the present disclosure.

### FIGURES:

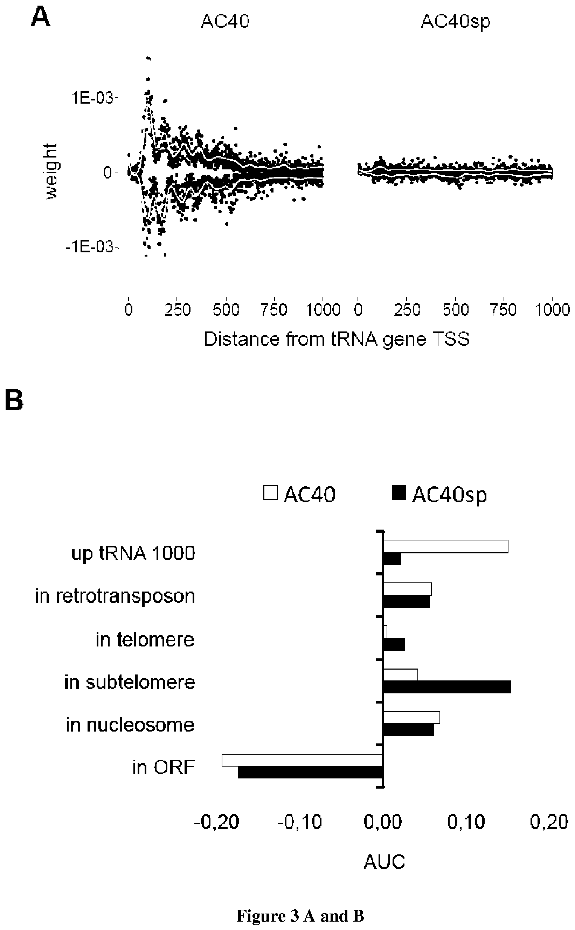

**Figure. 1****. Ty1 IN interacts with AC40 but not with its *S. pombe* ortholog, AC40sp.**
   (A) Two-hybrid interaction between different domains of Ty1-IN and AC40 or AC40sp. Left, Ty1 structure. Two open reading frames, *GAG* and *POL* (historically *TYA* and *TYB*), are flanked by two LTRs (black triangles). Protease (PR), integrase (IN), reverse transcriptase-RNAse H (RT/RH). The left-right arrow indicates Ty1 sequences recovered in the two-hybrid screen (2-HS) with AC40 as bait (coordinates 2706-4342 in Ty1). Ty1-IN regions fused to Gal4 activating domain (GAD) are depicted. Right, serial 10-fold dilutions of liquid cultures of cells expressing AC40 or AC40sp fused to Gal4 binding domain (GBD) and the various GAD-IN proteins were plated onto non-selective (Control) or selective (Interaction) media to detect interactions. (B) Co-immunoprecipitation of AC40 and IN-Strep. A 6xHA epitope was inserted at the end of the endogenous *RPC40* gene (AC40-HA) and IN was fused with streptavidine (IN-Strep) and expressed from a galactose inducible promoter. Immunoprecipitation of protein extracts was performed with anti-HA magnetic beads coupled to IgG. Proteins were revealed on western-blots with 12CA5 anti-HA and anti-Strep antibodies. (C) Co-immunoprecipitation of AC40 and IN expressed from a galactose inducible functional Ty1 element. Immunoprecipitation conditions are as in Fig. 3B. Proteins were revealed with 12CA5 anti-HA and 8B11 anti-IN antibodies.

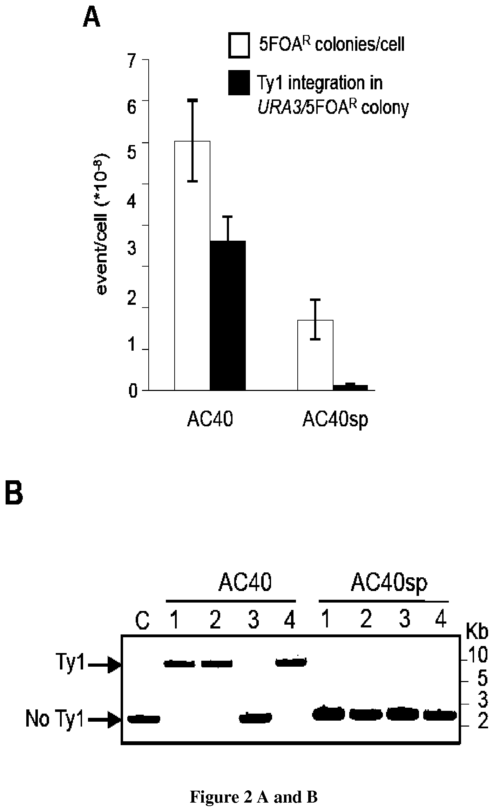

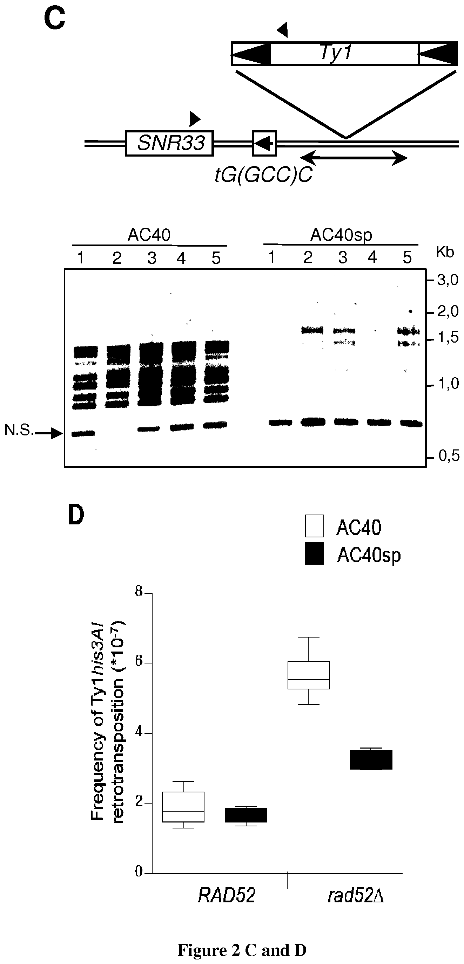

**Figure 2****. The interaction between Ty1-IN and AC40 is important for Ty1 targeting upstream two tRNA genes but not for Ty1 overall mobility.**
   (A) Ty1 integration into the *URA3-tG(GCC)B* locus. Histogram shows the average rate of 5FOA^{R} colonies obtained from cultures (32 and 55 for AC40 and AC40sp expressing strains, respectively) and induced for Ty1 retrotransposition at 20°C (grey bar) and of Ty1 insertions in *URA3*/5FOA^{R} colony identified by PCR (black bars) with standard errors. (B) PCR analysis of 5FOA^{R} colonies from independent cultures (30 and 27 for AC40 and AC40sp expressing strains, respectively) to detect Ty1 insertion events in *URA3* (primers indicated by leftwards and rightwards black triangles). Representative PCR results from 4 colonies for each strain are shown, a 6-kb band indicates a Ty1 element, a 2,5-kb band no insertion. A negative control (C) is shown: *URA3-tG(GCC)B* locus of a strain grown at the 30°C non permissive temperature for Ty1 retrotransposition. (C) Detection of Ty1 *de novo* insertions upstream of *tG(GCC)C* in AC40 and AC40sp expressing strains. Top: *tG(GCC)C* locus and experimental design. DNA was prepared from 5 independent cultures of each strain grown at 20°C and expressing Ty1 from the galactose inducible *GAL1* promoter. PCR reactions were carried out with primers specific for *tG(GCC)C* and Ty1 (leftwards and rightwards black triangles). Ty1 insertion occurs with equal frequency in both orientations (3, 4). Only insertion events in opposite orientation to *tG(GCC)C* were analyzed by PCR. N.S. a non specific band corresponding to the amplification of a fragment from adjacent Ty1 and Ty2 elements in the genome. (D) Frequency of Ty1*his3AI* retrotransposition in AC40 and AC40sp expressing strains, indicated as the number of His⁺ prototrophs divided by the total number of cells. Data from 6 independent cultures are represented as box plots.

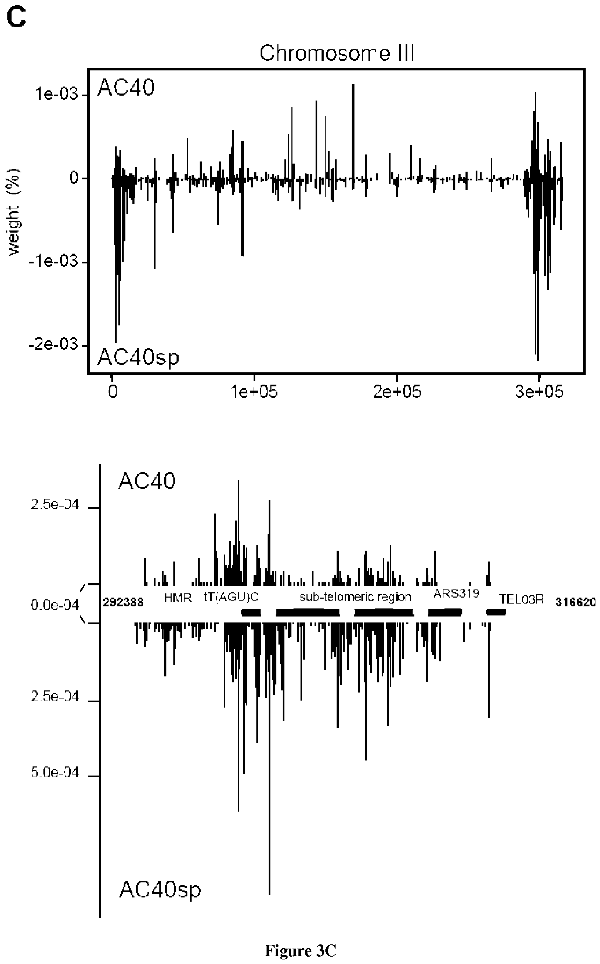

**Figure 3****. Association of Ty1 insertions with chromosomal features in WT (AC40) and loss-of-interaction mutant (AC40sp) strains.**
   (A) Ty1 insertions upstream of the 275 tRNA genes of *S. cerevisiae* were aggregated into a single distribution measuring distance to the start of transcription (position 0 on the x-axis). Black points above and below the x-axis denote convergent and divergent integrations, relative to the tRNA gene, respectively. LOESS (Locally weighted scatterplot smoothing) curves in red and blue indicate the general trends. The grey backdrop indicates the level of nucleosome at each position, based on the sequencing of chromosomal DNA after digestion by micrococcal nuclease (*30*)*.* The y-axis scale is normalized to the number of total integrations in each sample. The nucleosomes were linearly scaled for visibility of the periodicity. Left panel shows Ty1 insertions in WT (AC40) and right panel, Ty1 insertions in mutant (AC40sp). (B) Graphic of a subset of representative features with positive and negative AUC - 0.5 (AUC, Area Under the Curve values of the Receiver Operator Characteristic). 0 indicates a model of no predictive power, values of magnitude 0.5 indicate perfect prediction. Negative and positive values denote a feature that is associated with the control and with integration, respectively. Nucleosome AUC values were obtained from chromosomal regions for which nucleosomes data are available, the other AUC values were obtained from the entire genome dataset. (C) Top: Distribution of Ty1 insertions on chromosome III. Vertical bars indicate the number of unambiguous integrations at a particular site. Red and blue bars above and below the x-axis indicate insertions in WT (AC40) and loss of interaction mutant (AC40sp), respectively. Vertical lines indicate tRNA genes in forward (blue) or reverse (pink) orientations. Bottom: Zoom of the distribution of Ty1 insertions on the right subtelomere of chromosome III (coordinate 292388 to 316620). Depicted are the *HMR* locus (green thick line), the tT(AGU)C tRNA gene (light brown triangle), a large subtelomeric region containing non-essential genes (grey thick line), an ARS (blue thick line) and the telomere TEL03R (black thick line). X-axis denotes position along the chromosome at 1000-bp resolution for the whole chromosome and 100-bp for the right subtelomere. Y-axis scale shows percentage of total integrations in the genome per bin.

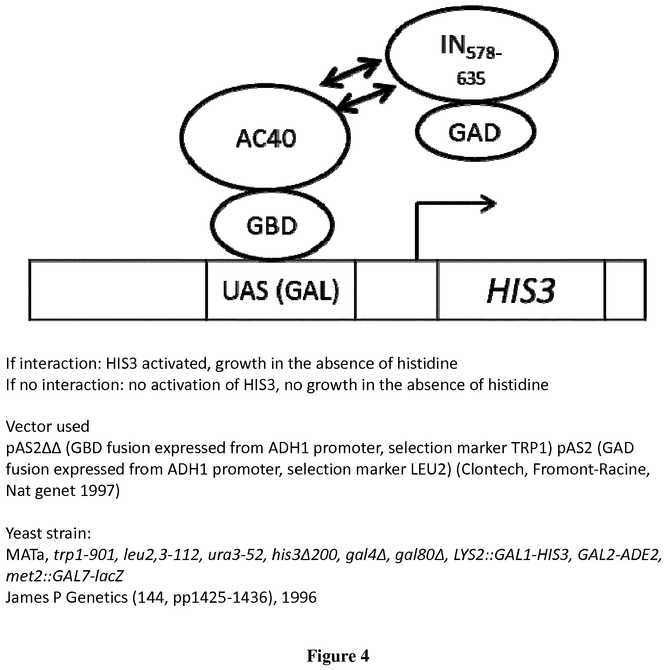

**Figure 4****: Scheme of the two-hybrid system.** Eukaryotic transcription factors consist of two main domains that are folded independently: a DNA binding domain (BD) that binds specific target sequences on a promoter and an activation domain (AD) that recruits the transcription machinery. The basic concept of our yeast two-hybrid screen is based on the reconstitution of an active transcription factor resulting from the interaction between two proteins, the "bait" protein, here AC40, fused to the BD of GAL4 (GBD), and the other protein, the "prey", here the different IN constructs fused to the AD of GAL4. The basis of the two-hybrid assay is that the transcription of the HIS3 reporter gene correlates to the interaction between the bait and prey proteins.

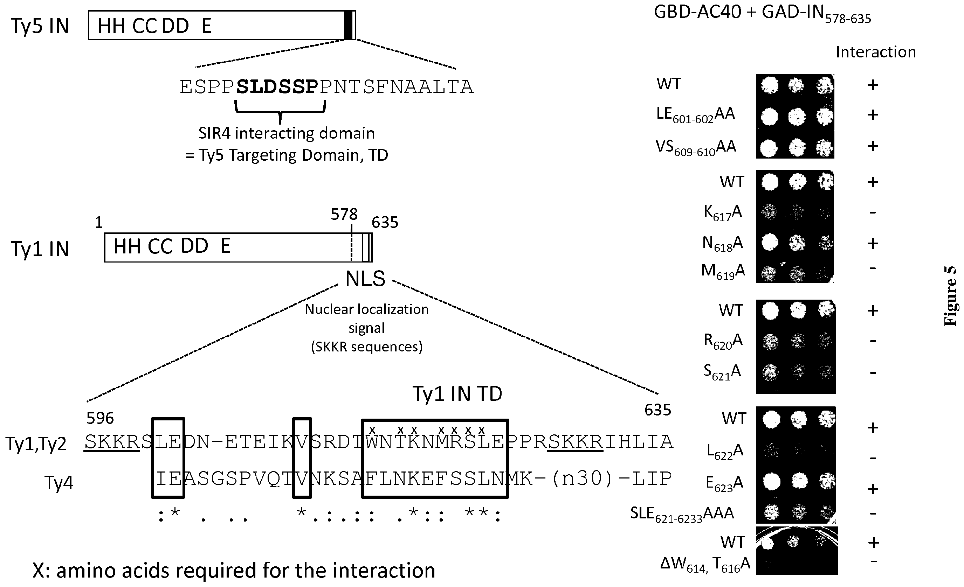

**Figure 5****: Characterization of Ty1 TD by two-hybrid assay.** On the top, Ty5 TD is depicted as an example. It was the first targeting domain identified in the integrase of a retrolelement. It corresponds to the minimal region interacting with the Sir4 protein, which targets Ty5 integration into heterochromatin regions. Ty1 TD was predicted on the basis of the alignments of the C-terminal region Ty1, Ty2 and Ty4 integrases because the three retrotransposons have the same preference to integrate upstream of Pol III transcribed genes. The rectangles indicate regions/amino acids that were well conserved between the 3 integrases. Alanine substitution of LE601-602, VS609-610, T616 (together with the deletion of W614), K617, N618, M619, R620, S621, L622, E623 and SLE621-623 was performed on GAD-IN578-635 and the interaction of the different fusion proteins with GBD-AC40 was tested. Growth indicates an interaction. (+) indicates an interaction between IN and AC40, (-) no interaction.

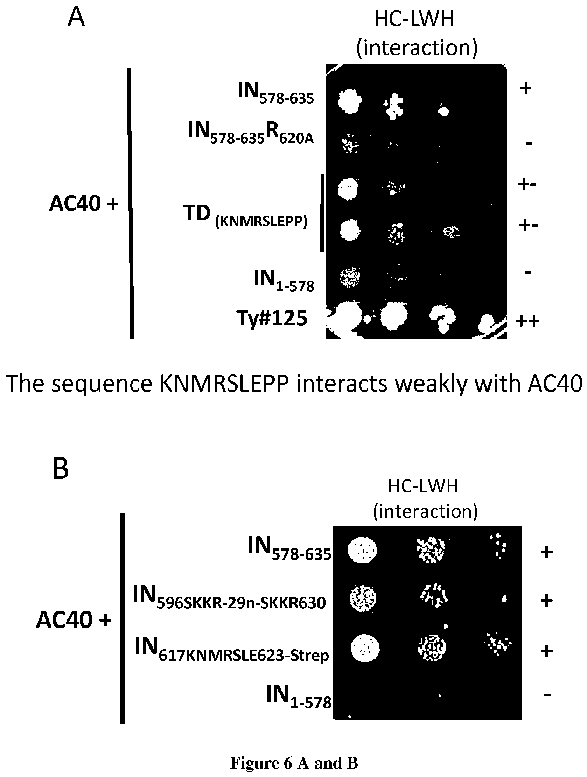

**Figure 6****: Characterization of the minimal Ty1 TD by two-hybrid assay. A)** The peptide 617KNMRSLEPP625 (SEQ ID NO: 7) of Ty1 IN was fused to GAD and the interaction between the two protein fusions were tested by two-hybrid assay. Positive control: IN₅₇₈₋₆₃₅, negative control: IN₅₇₈₋₆₃₅ with the R620A substitution, Ty#125: positive control, which corresponds to a sequence obtained in the original two-hybrid screen described in Example 1. Results indicate that the peptide 617KNMRSLEPP625 interacts weakly with AC40. **B)** The sequences IN₅₉₆₋₆₃₀ and IN₆₁₇₋₆₂₃ (SEQ ID NO: 4) fused to the streptavidine tag sequence interact with AC40. Positive control: IN₅₇₈₋₆₃₅, negative control IN₁₋₅₇₈.

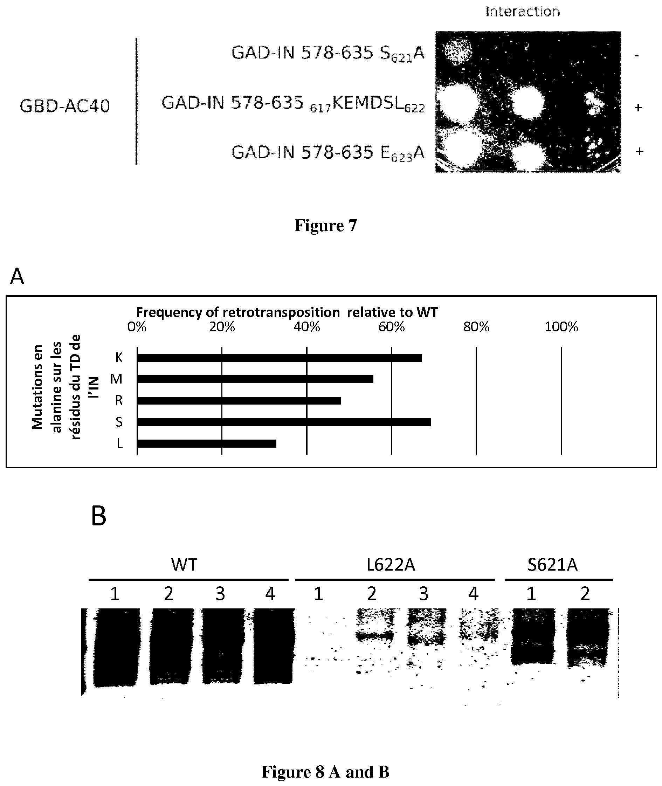

**Figure 7****: Replacement of the sequence 617KNMRSL622 by the sequence 617KEMDSL622 (SEQ ID N: 2) in GAD-IN578-635 maintains the interaction with GBD-AC40.** Interaction between GBD-AC40 and the different GAD-IN protein constructs were tested by two-hybrid assay. Negative control (GAD-IN578-635 with the S621A substitution), positive control, GAD-IN578-635 with the E623A substitution).
**Figure 8****: Mutations that abolish IN/AC40 interaction decrease Ty1 integration upstream of a Pol III transcribed genes but not Ty1 overall mobility.**
   **A) Ty1 overall mobility decreases no more than two-fold in Ty1 mutants carrying mutations that abolish IN/AC40 interaction.** Relative frequency of Ty1 retrotransposition in yeast strains expressing various mutants of Tylhis3AI, in which either of the K617, M619, R619, S621 or L622 residues where modified in A (alanine). For each strain, the number of His⁺ cells was divided by the total number of cells. Values were set at 100% of relative retrotransposition for WT Ty1 and normalized for Ty1 mutant elements.
   **B) Ty1 integration upstream of a Pol III genes is severely affected in Ty1 mutants carrying mutations that abolish IN/AC40 interaction.** Detection of Ty1 insertion events upstream of *tG(GCC)C* in yeast strains expressing various mutants of Ty1 (in which either of the S621 or L622 residues where modified in A (alanine)). A periodic banding pattern is characteristic of a functional Ty1 element (WT). Loss of this pattern in mutants indicates that they have lost the ability to integrate upstream of Pol III genes. The loss of this pattern was also obtained in mutants of Ty1 in which either of the K617 or M619 residues where modified in A (alanine). Experimental design is given in the legend of Fig 2C.

### EXAMPLES:

### EXAMPLE 1: An RNA polymerase III subunit determines sites of retrotransposon integration

A balance exists between the short-term detrimental effects of transposable elements as mutagens and the long-term, positive role they play as agents of genome plasticity and evolution (*1*). Important in maintaining this balance is integration site choice (ISC). Integration is typically not random and often occurs in regions of the genome where insertion is benign. For the retrotransposons - obligate genomic parasites - integration preferentially occurs in regions that are gene poor, likely the consequence of selection for mechanisms that favor non-deleterious insertions. Ty1, the most active and abundant LTR-retrotransposon of *S. cerevisiae,* preferentially integrates within a 1-kb window upstream of Pol III-transcribed genes (2). Ty1 targeting requires a functional Pol III promoter in the target gene (*2-4*) and is influenced by the chromatin-remodeling factor Isw2 and the Bdp1 subunit of TFIIIB (*5, 6*). However, the primary determinant of Ty1 ISC and its contribution in protecting the genome from deleterious insertions is still unknown.

In an attempt to identify protein interactors of Pol III, we performed systematic yeast two-hybrid screens using the 17 Pol III subunits as baits and a library of yeast DNA as prey. We identified a specific interaction between the AC40 subunit and a region of the Ty1 TyB/POL protein that encompasses the C-terminus of integrase (IN) and the N-terminus of reverse transcriptase (Fig. 1A). The screen also recovered Ty2 and Ty4 overlapping the same protein domains as Ty1, consistent with similar integration preferences for these 3 elements (7). HA-tagged AC40 (AC40-HA) co-immunoprecipitated IN when ectopically expressed either as a streptavidine-fusion (IN-Strep) (Fig. 1B) or from a functional Ty1 element (Fig. 1C).

Ty1 IN has two phylogenetically conserved regions (an N-terminal zinc-binding domain and a catalytic core with the DDX₃₅E motif) and a less conserved C-terminus containing a bi-partite nuclear localization signal required for IN nuclear localization and efficient retrotransposition (Fig. 1A) (8, 9). A two-hybrid assay confirmed the interaction between AC40 and full-length IN (IN₁₋₆₃₅) and revealed that the IN N-terminus and catalytic core were dispensable (IN₁₉₃₋₆₃₅), whereas the last 57 amino acids of the IN C-terminus were necessary and sufficient (Fig. 1A, IN₁₋₅₇₈ versus IN₅₇₈₋₆₃₅). The integrases of several retrotransposons and retroviruses interact with a cellular DNA-bound protein to mediate ISC (*10-16*). Similar to the yeast retrotransposon Ty5 and murine leukemia virus (MLV), the Ty1 targeting domain is located at the IN C-terminus, suggesting in some cases this region evolved to interact with specific targeting cofactors.

Since AC40 is an essential protein, we addressed its role in Ty1 ISC by using the *Schizosaccharomyces pombe rpc40*⁺ ortholog of *RPC40,* which encodes AC40. The *S. pombe* AC40 (AC40sp) could restore the viability of *S. cerevisiae* cells lacking AC40 and sustain normal Pol III transcription (*17*). However, GBD-AC40sp, when expressed at levels similar to GBD-AC40, did not show two-hybrid interaction with Ty1 IN (Fig. 1A and SID), implying that in its native state, AC40sp and Ty1 IN do not interact or only very poorly. Therefore, AC40sp was used as a loss-of-interaction mutant. We first determined the role of AC40 in Ty1 integration at Pol III-transcribed genes using a quantitative assay that relies on a *URA3* gene located upstream of the glycine tRNA gene *tG(GCC)B,* a hotspot of Ty1 integration. In this assay, Ty1 insertions upstream of *tG(GCC)B* inactivate *URA3,* resulting in Ura⁻ cells that grow on 5FOA plates. Endogenous Ty1 retrotransposition was induced in *rpc40*Δ strains expressing AC40 or AC40sp at similar levels by switching growth to 20°C, a temperature permissive for Ty1 retrotransposition. A ∼3.5-fold decrease in the number of 5FOA^{R} colonies per cell was observed in the presence of AC40sp (Fig. 2A). The fraction of 5FOA^{R} cells arising from Ty1 integration in cells expressing AC40sp was ∼30-fold lower than in AC40 expressing cells (Fig. 2A and 2B). Therefore, AC40/IN interaction is determinant for Ty1 integration at this reporter gene.

We further demonstrated the role of AC40 in Ty1 ISC by analyzing unselected insertions at the tRNA gene *tG(GCC)C,* another Ty1 integration hotspot, in strains that express a galactose-inducible Ty1 element (Fig. 2C). In the presence of AC40, we observed a ∼70-bp periodic banding pattern characteristic of Ty1 insertion in nucleosomal DNA upstream of tRNA genes (*3*, *4, 18*). In cultures expressing AC40sp, the pattern was dramatically altered, showing both a significant decrease in Ty1 integration and an apparent relaxed periodicity between hotspots (Fig. 2C). The same alteration in the pattern of integration at the *tG(GCC)C* locus was observed in AC40sp-expressing cells for endogenous Ty1 elements. Hence, AC40/IN interaction is critical for Ty1 integration specificity upstream of Pol III-transcribed genes.

To establish whether Ty1 integration frequency depends on the AC40/IN interaction, we used a chromosomal Ty1 element marked with a *his3AI* reporter gene, which confers His⁺ prototrophy to cells upon retrotransposition. Frequencies of His⁺ cells generated in strains expressing AC40 or AC40sp were similar (Fig. 2D). It is conceivable that in the loss-interaction mutant an overrepresentation of Ty1*HIS3* insertions could be due to homologous recombination (HR) with endogenous elements (*19*). We determined the frequency of IN-dependent Ty1*HIS3* insertion events in a *rad52*Δ mutant, which is deficient in HR (Fig. 2D). His⁺ events were slightly higher in *rad52*Δ mutant cells, consistent with previous studies showing that although the Rad52 pathway contributes to Ty1 HR, it globally inhibits Ty1 retrotransposition (*20*). Notably, the frequency of IN-dependent integration is 2-fold lower in AC40sp cells compared to AC40 cells, suggesting that a higher proportion of Ty1 insertions might be due to recombination in the absence of interaction. However, this slight decrease can not explain the dramatic effect observed on Ty1 integration at the *URA3-tG(GCC)B* reporter gene. Altogether, these results indicate that the AC40/IN interaction is required for the canonical Ty1 targeting pattern upstream of two representative tRNA genes, but the interaction does not influence integration efficiency. Therefore, the interaction with AC40 could function specifically to restrict Ty1 integration upstream of Pol III-transcribed genes to prevent potentially harmful insertions at secondary targets in the genome.

We compared the genome-wide integration patterns of Ty1 in the WT and AC40sp loss-of-interaction mutant strains. The WT strain exhibited a typical integration pattern at Pol III-transcribed genes (*3, 4*) (Fig. 3A). On the contrary, the loss-of-interaction mutant displayed a fundamentally different profile with only a slight bias to integrate near tRNA genes. Residual interaction between IN and AC40sp could explain this modest bias. To determine if other genomic features influence Ty1 ISC in the absence of the AC40/IN interaction, we used single (Fig. 3B) and multi-dimensional logistic regression models to associate genomic features with integration hotspots. Ty1 insertions in the WT strain were associated with upstream regions of tRNA genes or features associated with these sites, such as pre-existing retrotransposons (Fig. 3B). Regions with well-positioned nucleosomes were also favored and open reading frames (ORFs) avoided. In the AC40sp loss-of-interaction mutant, tRNA genes were no longer the primary targeting determinant but nucleosomes were still favored. Telomeres and subtelomeric regions were also preferred targets in the WT and even more so in the mutant (Fig. 3B, and 3C). This propensity for insertions to occur in heterochromatic domains was observed for all chromosomes. Noteworthy, Ty5, a related yeast retrotransposon, also targets heterochromatin by specifically interacting with the heterochromatin protein Sir4 (*11*). Thus, an interaction between Ty1 IN and heterochromatin proteins could be involved in Ty1's secondary target site preference.

We identified an interaction between Ty1 IN and the Pol III subunit AC40 that determines Ty1 ISC upstream of Pol III genes. Previous studies indicated that Ty1 integration requires a functional Pol III promoter, and our characterization of AC40 as a cofactor of Ty1 integration explains this requirement. The retroelements Ty3 of *S. cerevisiae* and TRE5-A of *Dictyostelium discoideum* also integrate at Pol III-transcribed genes, but their targeting involves an interaction with TFIIIB (*21, 22*). Our study demonstrates a direct role of an RNA polymerase in integration targeting. In Pol III, AC40 is located at the periphery of the complex in close proximity to the upstream DNA region (*23*). This location might explain why Ty1 integrates mostly upstream of Pol III transcribed-genes and rarely downstream. Ty1 does not integrate near or into genes transcribed by Pol I, which also contains AC40 (*3, 4*), The AC40/IN interaction could require additional cofactor(s) specific to Pol III transcription. Noteworthy, AC40 in Pol III is in close contact with TFIIIB (*24*), which contains Bdp1, a protein required for the Isw2-dependent periodic integration of Ty1 (6). This cofactor could participate directly or indirectly to the interaction. Ty1 integrates preferentially in nucleosomes, and the chromatin at Pol III promoters is relatively open structure with well-organized nucleosomes (*25, 26*). This organization is not found at Pol I genes, which are either actively transcribed or packed into a tight and repressive nucleosomal structure (*27*) - chromatin states that might disfavor Ty1 integration.

The Pol III transcription complex, which includes AC40, is regulated by nutrient and stress signaling pathways (*28*). As a result, Ty1 exhibits the potential to act as a mutagenic agent under conditions that affect AC40/IN interaction or Pol III transcription. tabOur work reveals that Ty1 also targets subtelomeres, especially in the absence of AC40/IN interaction. Yeast chromosome ends contain non-essential fast-evolving gene families generally needed to respond to environmental changes (*29*). Therefore, targeting Ty1 integration to subtelomeres could further protect the yeast genome from Ty1 mobility, while potentially promoting evolutionary adaptation and gene innovation in response to stress.

Understanding the molecular mechanism of targeted integration of the Ty1 retroelement has been a Holy Grail of mobile genetic element research for decades. With our study, we elucidate this molecular mechanism by indicating that the AC40 subunit of Pol III is the major cellular protein that targets Ty1 integration to Pol III genes, through its interaction with Ty1 integrase. Importantly, the tethering of integration complexes to the cell genome, through an interaction between the integrase and cellular proteins bound at favored insertion sites, is conserved with MLV and HIV retroviruses. The main difference is that MLV and HIV integrases interact with cellular factors that target retroviral integration in the promoter or transcription unit of Pol II transcribed genes, leading to potential gene deregulation or inactivation upon their integration.

The stable integration of retroelements into the host cell genome constitutes a major advantage for a retrovirus-based gene delivery system, especially for gene therapy, which aims at long-term correction of genetic defects, but also raises concerns about potential mutagenesis and oncogene activation. Gene therapy with MLV-derived retroviral vectors to treat severe immunodeficiency obtained impressive therapeutic success. However, the development of leukemia caused by insertional mutagenesis in some patients uncovered a serious unanticipated side effect due to the transactivation of adjacent oncogenes by the enhancer activity of the viral LTR. A key advance was the creation of more sophisticated "self-inactivating" (SIN) vectors, designed to avoid such transactivation events upon integration. However, these vectors still integrate near proto-oncogenes, maintaining a Damocles sword on the safety of such vectors. Therefore, a potential improvement for the future is to target gene therapy vectors to safe regions of the genome. Ty1 targeting mechanism upstream of individually non-essential Pol III genes provides safe landing sites that prevent deleterious consequences on cell fitness, and is therefore a promising alternative to already existing retroviral-based vectors designed for human gene therapy. Accordingly, the domain of Ty1 responsible for the interaction with the RNA polymerase III could thus be suitable to engineering chimeric integrases of retrovirus so as to drive the targeted integration of a transgene into safe regions of a eukaryotic genome, in order to reduce potential detrimental effect.

### EXAMPLE 2: Characterization of Ty1 IN Targeting Domain

We used the two-hybrid approach as depicted in Figure 4 to further characterize the TY1 domain responsible for the interaction with AC40: if the interaction occurs, then the HIS3 is activated and the growth can occur in the absence of histidine; if no interaction occurs then there is no activation of HIS3 and accordingly the growth cannot occur in the absence of histidine. Briefly, the experimental design was as follows: strain PJ69-4A is transformed with the 2 vectors pAS2ΔΔ and pACTII, containing either GBD-AC40 or any of the GAD-IN constructs tested in the examples. Cultures were grown overnight at 30°C. Serial 10-fold dilutions of aliquots of 1 DO600, washed in 1 ml of H20, were plated on HC medium lacking leucine and tryptophane (control) or leucine, tryptophane and histidine (interaction). Leucine and tryptophan prototrophies guaranty the presence of the 2 vectors. Plates were incubated 3 days at room temperature. As shown in Figure 5, the minimal sequence responsible for the interaction of IN578_635 with AC40 is the sequence which ranges from the amino acid residue at position 617 to 623. We also demonstrated that the amino acid residues at position 618 or 623 can be substituted. In Figure 6 we showed that the sequence KNMRSLEPP can also interact with AC40. In figure 7, we replaced in GAD-IN578-635, the sequence 617KNMRSL622 by KEMDSL (the amino acids that were modified are underlined) to demonstrate that the sequence KEMDSL also interacts with AC40.

### REFERENCES:

Throughout this application, various references describe the state of the art to which this disclosure pertains.
1. H. L. Levin, J. V Moran, Nat Rev Genet 12, 615-627 (2011).
2. S. E. Devine, J. D. Boeke, Genes Dev. 10, 620-633 (1996).
3. J. A. Baller, J. Gao, R. Stamenova, M. J. Curcio, D. F. Voytas, Genome Res. 22, 704-13 (2012).
4. L. Mularoni et al., Genome Res 22, 693-703 (2012).
5. M. E. Gelbart, N. Bachman, J. Delrow, J. D. Boeke, T. Tsukiyama, Genes Dev 19, 942-954 (2005).
6. N. Bachman, M. E. Gelbart, T. Tsukiyama, J. D. Boeke, Genes Dev 19, 955-964 (2005).
7. J. M. Kim, S. Vanguri, J. D. Boeke, A. Gabriel, D. F. Voytas, Genome Res. 8, 464-478 (1998).
8. M. Kenna, C. Brachmann, S. Devine, J. Boeke, Mol Cell Biol 18, 1115-1124 (1998).
9. S. P. Moore, L. A. Rinckel, D. J. Garfinkel, Mol Cell Biol 18, 1105-1114 (1998).
10. J. Kirchner, C. M. Connolly, S. B. Sandmeyer, Science 267, 1488-1491 (1995).
11. W. Xie et al., Mol Cell Biol 21, 6606-6614 (2001).
12. Y.-E. E. Leem et al., Mol Cell 30, 98-107 (2008).
13. A. Engelman, P. Cherepanov, PLoS Pathog 4, e1000046 (2008).
14. J. De Rijck et al., Cell Rep. 5, 886-894 (2013).
15. S. S. Gupta et al., J. Virol. 87, 12721-36 (2013).
16. A. Sharma et al., Proc. Natl. Acad. Sci. U. S. A. 110, 12036-41 (2013).
17. G. V Shpakovski, E. K. Shematorova, Curr Genet 36, 208-214 (1999).
18. N. Bachman, Y. Eby, J. D. Boeke, Genome Res 14, 1232-1247 (2004).
19. G. Sharon, T. J. Burkett, D. J. Garfinkel, Mol Cell Biol 14, 6540-51. (1994).
20. A. J. Rattray, B. K. Shafer, D. J. Garfinkel, Genetics 154, 543-56. (2000).
21. L. Yieh, G. Kassavetis, E. P. Geiduschek, S. B. Sandmeyer, J Biol Chem 275, 29800-29807 (2000).
22. T. Chung, O. Siol, T. Dingermann, T. Winckler, Mol. Cell. Biol. 27, 8492-501 (2007).
23. C. Fernández-Tornero et al., Nature 502, 644-9 (2013).
24. A. Vannini, P. Cramer, Mol. Cell 45, 439-46 (2012).
25. K. Brogaard, L. Xi, J.-P. Wang, J. Widom, Nature 486, 496-501 (2012).
26. Y. Kumar, P. Bhargava, BMC Genomics 14, 402 (2013).
27. K. Merz et al., Genes Dev. 22, 1190-204 (2008).
28. R. D. Moir, I. M. Willis, Biochim. Biophys. Acta 1829, 361-75 (2013).
29. C. A. Brown, A. W. Murray, K. J. Verstrepen, Curr. Biol. 20, 895-903 (2010).
30. N. Kaplan et al., Nature 458, 362-366 (2009).

## Claims

1. A polypeptide which consists of the amino acid sequence selected from the group consisting of SEQ ID NO:2 to SEQ ID NO:12.

2. A chimeric integrase which comprises the polypeptide of claim 1 and which is selected from the group consisting of feline immunodeficiency virus (FIV) integrases, Foamy virus (FV) integrases, murine leukemia virus (MLV) integrases, and lentivirus integrases.

3. The chimeric integrase of claim 2 wherein the retroviral integrase has a sequence having at least 80% of identity with SEQ ID NO: 13 or SEQ ID NO:14.

4. The chimeric integrase of claim 2 wherein the polypeptide of claim 1 is fused at the C-terminal end of the integrase protein or at the N-terminal end of the integrase protein.

5. The chimeric integrase of claim 2 wherein the polypeptide of claim 1 is inserted in the sequence encoding for the integrase protein.

6. A nucleic acid molecule which encodes for a polypeptide of claim 1 or a chimeric integrase of claim 2.

7. A retroviral vector which comprises the chimeric integrase of claim 2.

8. The retroviral vector of claim 7 which derives from a retrovirus selected from the group consisting of alpharetroviruses, betaretroviruses, gammaretroviruses, deltaretroviruses, epsilonretroviruses, lentiviruses and spumaviruses.

9. The retroviral vector of claim 7 which comprises a transgene.

10. An in vitro method for expressing a transgene into the genome of a plurality of cells comprising infecting the plurality of cells with the retroviral vector of claim 7.

11. The retroviral vector of claim 7 for use in gene therapy.

## Patentansprüche

1. Polypeptid, das aus der Aminosäuresequenz besteht, die aus der Gruppe, bestehend aus SEQ ID NO:2 bis SEQ ID NO:12, ausgewählt ist.

2. Chimäre Integrase, die das Polypeptid nach Anspruch 1 umfasst und die aus der Gruppe, bestehend aus felinen Immundefizienzvirus-Integrasen (FIV), Foamyvirus-Integrasen (FV), murinen Leukämievirus-Integrasen (MLV) und Lentivirus-Integrasen, ausgewählt ist.

3. Chimäre Integrase nach Anspruch 2, wobei die retrovirale Integrase eine Sequenz aufweist, die zu mindestens 80 % mit SEQ ID NO:13 oder SEQ ID NO:14 identisch ist.

4. Chimäre Integrase nach Anspruch 2, wobei das Polypeptid nach Anspruch 1 am C-terminalen Ende des Integraseproteins oder am N-terminalen Ende des Integraseproteins fusioniert ist.

5. Chimäre Integrase nach Anspruch 2, wobei das Polypeptid nach Anspruch 1 in die Sequenz, die das Integraseprotein codiert, inseriert ist.

6. Nukleinsäuremolekül, das ein Polypeptid nach Anspruch 1 oder eine chimäre Integrase nach Anspruch 2 codiert.

7. Retroviraler Vektor, der die chimäre Integrase nach Anspruch 2 umfasst.

8. Retroviraler Vektor nach Anspruch 7, der von einem Retrovirus stammt, der aus der Gruppe, bestehend aus Alpharetroviren, Betaretroviren, Gammaretroviren, Deltaretroviren, Epsilonretroviren, Lentiviren und Spumaviren, ausgewählt ist.

9. Retroviraler Vektor nach Anspruch 7, der ein Transgen umfasst.

10. In-vitro-Verfahren zum Exprimieren eines Transgens in das Genom einer Vielzahl von Zellen, umfassend das Infizieren der Vielzahl von Zellen mit dem retroviralen Vektor nach Anspruch 7.

11. Retroviraler Vektor nach Anspruch 7 zur Verwendung bei der Gentherapie.

## Revendications

1. Polypeptide qui consiste en la séquence d'acides aminés sélectionnée dans le groupe consistant en SEQ ID NO : 2 à SEQ ID NO : 12.

2. Intégrase chimère qui comprend le polypeptide selon la revendication 1 et qui est sélectionnée dans le groupe consistant en les intégrases du virus de l'immunodéficience féline (VIF), les intégrases du virus spumeux (VS), les intégrases du virus de la leucémie murine (VLM), et les intégrases de lentivirus.

3. Intégrase chimère selon la revendication 2 dans laquelle l'intégrase rétrovirale présente une séquence présentant au moins 80 % d'identité avec SEQ ID NO : 13 ou SEQ ID NO : 14.

4. Intégrase chimère selon la revendication 2 dans laquelle le polypeptide selon la revendication 1 est fusionné à l'extrémité C-terminale de la protéine d'intégrase ou à l'extrémité N-terminale de la protéine d'intégrase.

5. Intégrase chimère selon la revendication 2 dans laquelle le polypeptide selon la revendication 1 est inséré dans la séquence codant pour la protéine d'intégrase.

6. Molécule d'acide nucléique qui code pour un polypeptide selon la revendication 1 ou une intégrase chimère selon la revendication 2.

7. Vecteur rétroviral qui comprend l'intégrase chimère selon la revendication 2.

8. Vecteur rétroviral selon la revendication 7 qui dérive d'un rétrovirus sélectionné dans le groupe consistant en les alpharétrovirus, les bêtarétrovirus, les gammarétrovirus, les deltarétrovirus, les epsilonrétrovirus, les lentivirus et les spumavirus.

9. Vecteur rétroviral selon la revendication 7 qui comprend un transgène.

10. Procédé in vitro d'expression d'un transgène dans le génome d'une pluralité de cellules comprenant l'infection de la pluralité de cellules avec le vecteur rétroviral selon la revendication 7.

11. Vecteur rétroviral selon la revendication 7 pour une utilisation en thérapie génique.
